# DEMANDE DE BREVET EUROPEEN

(11) **EP 1 092 436 A1**
(43) Date de publication de la demande: **18.04.2001**
(21) Numéro de dépôt: 00402788.4
(22) Date de dépôt: 10.10.2000
(51) Int. Cl.: A61K 35/32, C12N 5/08, C12N 5/10, A61K 48/00

(54) **Compositions de ténocytes, préparation et utilisations**

(30) Priorité: 12.10.1999 FR 9912711
(71) Demandeur: UNIVERSITE PIERRE ET MARIE CURIE PARIS VI, 75252 Paris Cédex 05 (FR)
(72) Inventeur: Saillant, Gérard, 78170 La Celle Saint Cloud (FR); Klatzmann, David, 75013 Paris (FR); Salzmann, Jean-Loup, 75005 Paris (FR); Boggione, Christophe, 75007 Paris (FR); Boyer, Olivier, 75018 Paris (FR); Jayankura, Marc, 1170 Bruxelles (BE)
(74) Mandataire: Becker, Philippe

(57) **Abrégé**

La présente invention concerne des compositions de cellules ténocytes, notamment humaines, et des méthodes pour leur préparation. Elle concerne également l'utilisation de ces compositions pour l'implantation de ténocytes in vivo, pour le traitement de différentes pathologies. L'invention est plus particulièrement relative à la production de suspensions de ténocytes humains, notamment autologues, des techniques pour leur conservation, leur modification génétique ex vivo ou in vivo, et leur utilisation in vivo pour restaurer des structures ligamentaires ou tendineuses affectées ou, plus généralement, pour le traitement et la réfection de tout défaut cliniquement significatif des tendons, ligaments ou autres tissus biologiques comprenant des ténocytes ou une structure fibreuse.

## Description

La présente invention concerne des compositions de cellules ténocytes, notamment humaines, et des méthodes pour leur préparation. Elle concerne également l'utilisation de ces compositions pour l'implantation de ténocytes in vivo, pour le traitement de différentes pathologies. L'invention est plus particulièrement relative à la production de suspensions de ténocytes humains, notamment autologues, des techniques pour leur conservation, leur modification génétique ex vivo ou in vivo, et leur utilisation in vivo pour restaurer des structures ligamentaires ou tendineuses affectées ou, plus généralement, pour le traitement et la réfection de tout défaut cliniquement significatif des tendons, ligaments, muscles ou autres tissus biologiques comprenant des ténocytes ou une structure fibreuse.

Le tendon est essentiellement composé de 30% de fibres de collagène et de 2% de fibres d'élastine, enveloppées dans une matrice extra-cellulaire contenant 68% d'eau. Les différents composants du tendon sont :
- les fibres de collagène de type I : composante majoritaire constituant 70% du poids sec du tendon. Leur disposition, parallèle aux contraintes mécaniques, est responsable de la résistance du tendon aux forces de traction.
- les fibres d'élastine, qui assurent la part d'élasticité du tendon.
- la matrice extra-cellulaire, composée de protéoglycanes, de glycoprotéines et d'eau, et
- une composante cellulaire, rare, principalement représentée par les ténocytes (fibroblastes différenciés des tendons, aponévroses et ligaments).

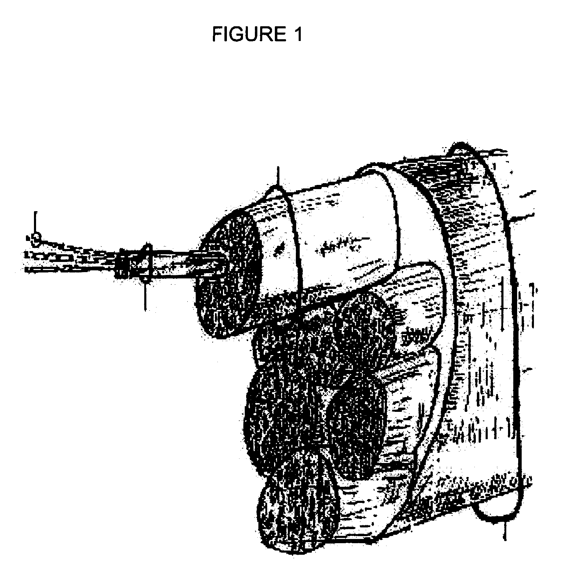

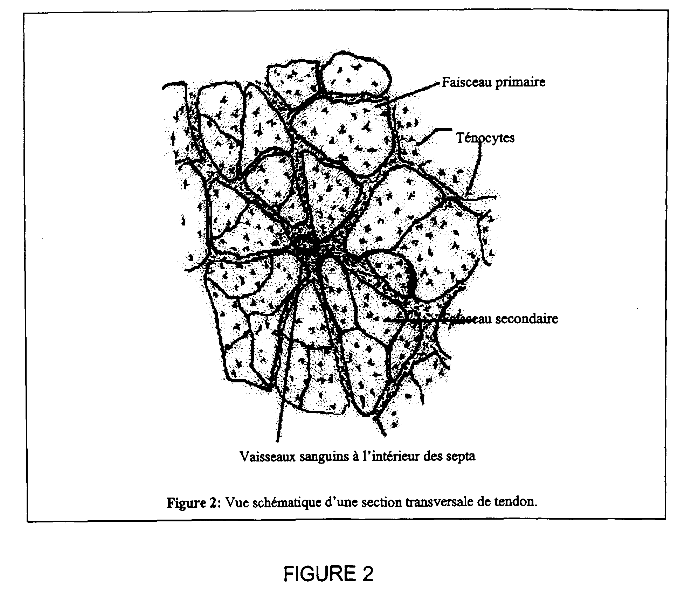

Le tendon est organisé en structures de taille croissante:
- Fibres de collagène : L'unité de base du collagène est constituée par le tropocollagène. Il s'agit d'une longue protéine de 200 nm sur 1,5 nm, principalement de type I, formée par l'assemblage de 3 chaînes alpha enroulées en triple hélice. Le tropocollagène résulte de la polymérisation de molécules de procollagène secrétées par les ténocytes. L'assemblage de 5 unités de tropocollagène forme une fibrille de collagène. Plusieurs fibrilles parallèles, enveloppées par la matrice extra-cellulaire, forment une fibre de collagène (Fig. 1).
- Faisceaux de collagène : Les fibres de collagène se regroupent en faisceaux disposés dans l'axe du tendon. Les faisceaux sont séparés par un tissu conjonctif lâche, l'endotendon, contenant les éléments vasculo-nerveux et permettant le glissement entre faisceaux (Fig. 2).
- Le tendon proprement dit : La réunion des faisceaux, primaires et secondaires, constitue le tendon enveloppé par une membrane conjonctive, l'épitendon (ou périténonium).

Autour du tendon, les annexes sont constituées par le paratendon, tissu conjonctif lâche assurant le soutien du tendon, ou par les gaines fibreuse et synoviale assurant le maintien et le glissement du tendon. La gaine synoviale est formée de deux feuillets constituant un espace de glissement.

La vascularisation du tendon provient principalement de ses extrémités. Elle est assurée par des artérioles musculaires et périostées. Il existe également un apport vasculaire provenant du mésotendon ou du paratendon dont l'importance est moindre. Ainsi, le tiers moyen du tendon, mal vascularisé, constitue une zone critique au plan vasculaire, ce qui pourrait rendre compte de sa fragilité mécanique particulière.

Le tendon, tout comme les ligaments, est un tissu relativement fragile, qui subit de multiples altérations, soit de nature traumatique, soit liées au vieillissement.

Ainsi, la structure du tendon se modifie physiologiquement avec l'âge et peut expliquer la fragilisation observée lors du vieillissement (1). Les facteurs de fragilisation tendineuse sont la diminution du nombre de ténocytes, la diminution du nombre de fibres élastiques, la diminution de la qualité puis de la quantité des fibres de collagène, ainsi que, la diminution de la quantité de protéoglycanes et, par conséquent, du niveau d'hydratation.

Parmi les facteurs exogènes aggravant la fragilisation tendineuse, on note les microtraumatismes répétés (des microruptures apparaissent au-delà de 4% d'étirement du tendon), la prise d'androgènes (par l'augmentation du rapport masse musculaire / tendon) et la prise de corticostéroïdes (par diminution de l'activité fibroblastique). La diminution, absolue ou relative, du nombre de ténocytes, ainsi que de leurs possibilités métaboliques, aboutit à une limitation de la régénération tendineuse. La faible vascularisation du tendon renforce ce phénomène.

Lors d'altérations des tendons ou ligaments, un processus naturel lent de cicatrisation se met en place, qui peut être schématisé en quatre phases.

Une première phase d'inflammation (72 heures), caractérisée par la rétraction des berges tendineuses, la formation d'un hématome dans l'espace inter-fragmentaire, la coagulation et la libération de cytokines (histamine, sérotonine, prostaglandines, bradykinine) initiant la phase inflammatoire. Survient ensuite, la phase cellulaire marquée par l'augmentation du nombre de macrophages puis par une prolifération des ténocytes (différenciation de cellules issues du paratendon). Il se produit alors la synthèse d'un collagène de type III (immature) de faible tenue mécanique.

Une deuxième phase, de prolifération cellulaire (6 semaines), caractérisée par l'organisation du caillot (apparition de néovaisseaux, dégradation macrophagique et réhabitation par des fibroblastes), par l'augmentation du nombre de ténocytes synthétisant du collagène de type I (mature) s'orientant selon les forces de contrainte et par l'augmentation de la quantité de protéoglycanes. La qualité mécanique devient satisfaisante à J45.

Une troisième et une quatrième phases de remodelage et maturation de la zone cicatricielle, caractérisées par la diminution progressive de la densité cellulaire du tendon, par l'augmentation de la densité en collagène de type I correctement orienté, par la diminution du volume cicatriciel et par l'augmentation de la résistance mécanique.

Le retour vers une structure proche de la normale demande un délai d'environ un an. Ce mécanisme de cicatrisation est donc très lent, souvent perturbé par les mouvements des tendons et/ou ligaments, et conduit souvent à des structures fragilisées, car ayant une composition fibreuse et cellulaire différente du tissu d'origine.

Dans le cas particulier de la cicatrisation après réalisation d'une autogreffe, on observe dans un premier temps, la disparition des fibroblastes du transplant et la résorption macrophagique du transplant, conduisant à une fragilisation de celui-ci. Puis apparaît une phase d'hypervascularisation avec des néovaisseaux suivie de la réapparition des ténocytes. Enfin, une phase de remodelage caractérisée par la synthèse de matrice extracellulaire et de collagène de type I permet une augmentation de la qualité mécanique. Le néotendon prend finalement une structure proche de l'ancien tendon sous l'effet des contraintes mécaniques. Cette transformation, constituant la phase de ligamentisation, s'étend sur une période de 2 ans environ (2).

Les approches thérapeutiques actuelles pour le traitement des altérations du tendon, qu'elles soient d'origine traumatique ou liées au vieillissement, sont :
- la cicatrisation dirigée du tendon, favorisée par une baisse de l'activité voire une immobilisation,
- la réparation du tendon par suture directe, parfois aidée d'un renfort synthétique (biomatériaux résorbables ou non),
- le remplacement du tendon par une greffe de tissu biologique (autologue ou allogénique) ou par un substitut synthétique.

Dans le premier cas, le résultat aboutit souvent à une guérison incomplète comportant une séquelle fibreuse. Dans les deux derniers cas, il existe de multiples problèmes de compatibilité biomécanique compromettant les résultats sur le long terme (3,4). Par ailleurs, la pauvreté cellulaire et l'insuffisance de vascularisation limitent la régénération tendineuse.

La présente invention propose à présent de nouvelles approches thérapeutiques pour restaurer des défauts du tendon ou d'autres tissus biologiques comprenant des ténocytes ou de type fibreux ou conjonctif. La présente invention repose notamment sur la mise au point de compositions et méthodes permettant d'augmenter la composante cellulaire du tendon et/ou sa vascularisation. La présente invention repose en particulier sur l'implantation de ténocytes in vivo ou sur des compositions permettant d'augmenter le nombre de ténocytes présents dans le tissu biologique in vivo, de manière à recoloniser et reconstituer la structure défectueuse.

La présente invention repose, d'une manière générale, sur l'utilisation de ténocytes pour des applications thérapeutiques, de reconstitution de tissu, notamment chez l'homme. L'invention repose notamment sur l'utilisation de ténocytes humains, en particulier autologues, multipliés in vitro, comme médicament susceptible d'agir pour la reconstitution de tissus biologiques, notamment de tissus biologiques fibreux ou conjonctif. Dans une application préférée, les ténocytes selon l'invention sont utilisés pour reconstituer des tendons ou ligaments présentant des défauts, comme par exemple les tendinites, déchirement, entorses, etc. Cette application est particulièrement avantageuse car permet, au contraire des processus de traitement actuels, de régénérer des tissus ayant les propriétés mécaniques et physiologiques proches des, voire similaires aux tissus naturels.

La présente invention décrit également des méthodes permettant de produire et de multiplier in vitro des ténocytes, à partir de prélèvements de tissus biologiques, notamment de prélèvements de fragments de tendons ou ligaments sains.

La présente invention montre en outre qu'il est possible d'implanter in vivo des ténocytes cultivés in vitro, le cas échéant après modification génétique de ceux-ci, et que ces ténocytes implantés sont viables, fonctionnels, et capables d'exprimer un produit recombinant. A cet égard, de manière générale, l'invention concerne toute méthode thérapeutique comprenant l'implantation dans un tendon ou un ligament de cellules modifiées génétiquement, par exemple de ténocytes ou de fibroblastes.

L'invention montre encore qu'il est possible de transférer in vivo des acides nucléiques recombinants dans des ténocytes pour leur faire exprimer des produits biologiques d'intérêt. La présente invention décrit donc, de manière générale, des approches thérapeutiques pour la reconstitution de tendons, ligaments ou autres tissus fibreux, basées sur l'implantation de ténocytes ou sur la modification in situ de ténocytes, notamment chez l'homme.

La présente invention sera décrite plus en détails dans la suite du texte. Pour une meilleure compréhension de la présente invention, les définitions suivantes sont fournies :

Ténocyte : Au sens de la présente invention, le terme « ténocyte » désigne les cellules de la composante cellulaire des tendons, ligaments ou aponévroses, par exemple, ou d'autres tissus de type fibreux. Bien que les ténocytes des tendons, ligaments ou aponévroses puissent présenter des caractéristiques physiologiques propres, il s'agit de cellules fusiformes à noyau fin, d'origine fibroblastique. En outre, ces cellules sont capables de produire le collagène, l'élastine et la matrice extra-cellulaire. Le terme ténocyte désigne, au sens de l'invention, aussi bien des cultures primaires que des cultures secondaires (obtenues par multiplication in vitro) ou des lignées établies.

Fibroblaste : Cellule fusiforme du tissu conjonctif, capable de synthétiser les protéines de la matrice extracellulaire.

Acide nucléique recombinant : Le terme acide nucléique recombinant désigne tout acide nucléique codant un produit d'intérêt, qu'il s'agisse d'un ARN, ou d'un polypeptide (ce terme désignant généralement toute protéine, fragment de protéine ou peptide). Il s'agit plus préférentiellement d'un polypeptide ayant une activité biologique, notamment d'un facteur de croissance. L'acide nucléique recombinant peut être un ADNc, un ADNg, un ADN synthétique ou semi-synthétique, un oligonucléotide, un ARN, etc. Il peut s'agir d'un acide nucléique d'origine humaine, végétale, virale, procaryote, artificielle, etc. L'acide nucléique recombinant peut par ailleurs comprendre des séquences régulatrices de la transcription, telles que promoteurs, terminateurs, enhanceurs, séquence de sécrétion, etc. En outre, l'acide nucléique recombinant peut être sous forme linéaire, circulaire, simple-brin ou double-brin. Il peut faire partie d'un plasmide, cosmide, phage, chromosome artificiel, virus, etc.

ADN nu : Une composition comprenant un acide nucléique recombinant, dépourvue d'agent facilitant la transfection, y compris de virus. Il s'agit typiquement d'un fragment d'acide nucléique ou d'un plasmide, en solution saline et/ou glucosée (par exemple à 5% glucose).

Cellule Modifiée génétiquement : Toute cellule contenant un acide nucléique recombinant tel que défini ci-avant. Il peut s'agir d'une cellule dans laquelle cet ADN recombinant a été introduit, ou d'un descendant d'une telle cellule.

Un premier objet de l'invention réside plus particulièrement dans une composition comprenant des ténocytes humains, autologues et/ou allogéniques. Il s'agit avantageusement d'une composition comprenant essentiellement des ténocytes humains, c'est-à-dire de préférence dont plus de 60% des cellules sont des ténocytes humains. Typiquement, dans une composition de ténocytes selon l'invention, au moins 80% des cellules sont de type ténocytes. La composition peut comprendre en outre d'autres types de cellules (fibroblastes, cellules endothéliales, etc), ou d'autres facteurs biologiques. Les compositions peuvent être en solution, ou congelées, comme il sera expliqué dans la suite du texte. Lorsque qu'il s'agit de solutions (ou suspensions), les compositions peuvent comprendre un milieu de culture, un milieu de conservation et/ou d'injection. Par ailleurs, les compositions cellulaires selon l'invention peuvent être comprises dans tout dispositif (ou conteneur) approprié, par exemple une ampoule, seringue, fiole, poche, boite, etc.

Un autre objet particulier de l'invention réside dans une composition comprenant des ténocytes équins autologues et/ou allogéniques. Comme il sera expliqué dans la suite du texte, de telles compositions sont particulièrement avantageuses pour le traitement des affections des tendons et/ou ligaments des chevaux.

Un autre objet de l'invention réside dans une culture in vitro ou ex vivo de ténocytes humains ou équins, notamment de ténocytes humains autologues et/ou allogéniques. Il peut s'agir de ténocytes primaires ou multipliés in vitro ou ex vivo, le cas échéant obtenus à partir de banques établies au préalable. Les cultures comprennent avantageusement un milieu adapté à la culture ou la multiplication des cellules.

L'invention réside aussi dans une méthode de préparation de ténocytes, notamment humains, comprenant le prélèvement d'un fragment de tissu biologique comprenant des ténocytes, le traitement de ce fragment pour dissocier les ténocytes, et la mise en culture des ténocytes.

Dans un mode préféré de mise en oeuvre, le fragment de tissu biologique provient d'un tissu sain. Plus préférentiellement, il s'agit d'un petit fragment de tissu, par exemple d'un poids inférieur ou égal à 10g, préférentiellement inférieur ou égal à 1g, plus généralement compris entre 0,5 g et 5 g. La présente invention montre en effet qu'il est possible, à partir d'échantillons de taille restreinte, comportant un nombre faible de cellules ténocytes, d'isoler ces cellules et de les expandre in vitro.

Compte tenu de la faible cellularité du tendon et de la nécessité de prélever un explant le plus petit possible, dans l'optique d'une application à une situation de manque de substance d'un tendon lésé, la présente invention décrit la possibilité et les conditions d'obtention de ténocytes à partir de petits explants tendineux. L'extraction de ténocytes à partir d'un fragment de tendon de 150mg (tendon rotulien de lapin) a permis l'obtention d'une quantité de ténocytes de l'ordre de 10⁶ éléments dans un délai de 21 jours. La croissance est ensuite exponentielle avec un temps de doublement semblant diminuer avec le nombre de passages. A cet égard, l'invention montre également qu'une multiplication (expansion) importante des ténocytes en culture peut être obtenue en plusieurs passages successifs, lorsque les cellules sont ensemencées à environ 25% de la confluence. Cette caractéristique constitue un mode préféré du procédé de préparation de la présente invention. La digestion d'un tendon rotulien pesant 0,5g a permis l'obtention, en 24 heures, de 7.10⁵ cellules. Par extrapolation, ces expériences indiquent que à partir de 1g de tendon humain environ, il est possible de produire 10⁷ à 10⁸ cellules après 2 semaines de culture environ.

L'expérience montre aussi que les ténocytes multipliés in vitro conservent leur capacité à reconstituer du tendon, quand ils sont réimplantés in vivo.

Le prélèvement de l'échantillon de tissu peut être réalisé selon différentes techniques, comme par exemple la microdissection, la microlacération, au cours d'un peignage, la ponction intratendineuse ou intraligamentaire, le prélèvement d'un fragment d'aponévose, etc. Le fragment de tissu biologique peut également être obtenu par exemple à partir d'une banque.

Préférentiellement, le fragment de tissu biologique est un fragment de tendon, de ligament ou d'aponévrose. L'invention concerne donc des compositions de ténocytes obtenus (et/ou dérivés) à partir de tendon, de ligament ou d'aponévrose. Le fragment (contenant les ténocytes) peut être prélevé par des techniques chirurgicales ou microchirurgicales. A cet égard, il est également possible d'établir des banques de ténocytes, par exemple à partir de tout tissu tendineux ou ligamenteux prélevé chez un sujet (humain ou animal). Ces banques peuvent être conservées et éventuellement traitées pour permettre leur utilisation. Ces banques permettent de produire, à tout moment, des quantités importantes de ténocytes, par exemple autologues, et de disposer de telles cellules de manière rapide, sans qu'il soit nécessaire de procéder à des prélèvements, dissociation et/ou cultures lorsqu'une intervention doit être effectuée. L'invention concerne donc également un procédé de préparation de banques de ténocytes, comprenant l'obtention de ténocytes à partir de tissu biologique, leur multiplication in vitro, et leur conservation, le cas échéant après vérification des caractères de stérilité, absence de contamination, pureté, etc. Il peut s'agir de banques de ténocytes humains ou animaux, notamment équin. Ces banques peuvent être réalisées avantageusement de manière préventive pour des sujets présentant des risques de développer des lésions des ligaments, tendons ou muscles, comme par exemple les sportifs. Ces banques peuvent être conservées sous forme congelée, dans les conditions décrites ci-après.

De préférence, les ténocytes humains selon l'invention sont préparés à partir du tendon d'Achille ou des tendons suivants :
- tendons de la patte d'oie : Muscles droit interne (M.Gracilis) et demi-tendineux (M.Semitendinosus). Ces tendons sont quasiment inutilisés par l'être humain et peuvent être prélevés intégralement. Ils peuvent ainsi permettre l'obtention par culture in vitro selon l'invention de 10⁶ à 10⁷ cellules environ.
- tendon du muscle petit palmaire (M. Palmaris Longus). Ce tendon peut également être prélevé intégralement. Il est possible d'obtenir ainsi un volume de tissu de l'ordre de 1 à 3 g environ.
- tendon du muscle plantaire grêle (M. Plantaris). Ce tendon peut également être prélevé intégralement. Il est possible d'obtenir ainsi un volume de tissu de l'ordre de 2 à 4 g environ.
- tendon du muscle péronier antérieur (M. Peronus Tertius). Ce tendon peut également être prélevé intégralement. Il est possible d'obtenir ainsi un volume de tissu de l'ordre de 2 à 3 g environ.
- Tendron quadricipital A partir de ce tendon, il est possible de prélever un fragment de tissu d'un volume de l'ordre de 1g, sans affecter son fonctionnement ou risque de fragilisation.
- tendon rotulien (Lig. Patellae) : A partir de ce tendon, il est possible de prélever un fragment de tissu d'un volume de l'ordre de 1g, sans affecter son fonctionnement ou risque de fragilisation.

Les ténocytes peuvent également provenir des ligaments suivants :
- Ligament acromio-coracoïdien (Lig. Coraco-acromiale)
- Ligament latéral interne du genou (Lig. Collaterale Tibiale)
ou être prélevés à partir d'aponévroses telles que :
- aponévrose fémorale (Fascia lata)
- aponévroses des muscles jumeaux (M. Gastrocnemii).

Préférentiellement, les ténocytes humains selon l'invention sont obtenus (ou dérivés) à partir de tendon. Les ténocytes ainsi préparés devraient en effet présenter des caractéristiques mécaniques et physiologiques intéressantes. Préférentiellement, le fragment de tissu est traité par :
- découpage mécanique, et/ou
- digestion enzymatique, par exemple en présence d'enzyme(s) capable(s) d'hydrolyser les molécules de collagène.

Le découpage mécanique permet de faciliter et/ou d'accélérer la dissociation des cellules présentes dans le fragment. Ce traitement mécanique peut être réalisé par exemple au moyen de ciseaux, pinces, scalpels, bistouris, tamisage sur grilles, etc. Préférentiellement, le traitement mécanique est réalisé jusqu'à obtention d'une préparation tissulaire composée de fragments de taille inférieure à environ 20 mm³, de préférence inférieure à 10 mm³.

L'échantillon biologique est ensuite soumis à un traitement en présence d'une composition comprenant au moins une enzyme capable d'hydrolyser le collagène, de préférence le collagène de type I ou, plus généralement, capable de dégrader un ou plusieurs constituants de la matrice extracellulaire tendineuse (composée principalement de collagène de type I, associé à des protéoglycans). Une enzyme préférée pour cette étape est la collagénase. La collagénase utilisée est préférentiellement d'origine bactérienne, comme par exemple la collagénase D, la collagénase A, la collagénase P, la collagénase de Vibrio alginolyticus (EP430635) ou encore la collagénase ColH de Clostridium histolyticus (J. Bact. 181 (1999) 2816). D'autres collagénases peuvent également être utilisées, comme des collagénases d'origine eucaryote, notamment mammifère, par exemple humaine. En particulier, on peut utiliser des collagénases (recombinantes) de mammifère, spécifiques du collagène de type I et/ou du tendon ou également des collagénases modifiées, par exemple par mutagénèse dirigée, possédant des propriétés améliorées en terme d'efficacité, de sélectivité, de conditions d'activité, etc. En particulier, il peut s'agir de collagénases modifiées (par exemple par mutagénèse dirigée), capables d'exercer une activité à des températures basses, notamment inférieures à 20°C, plus préférentiellement inférieures à 10°C.

Dans un mode particulier, il s'agit d'une collagénase recombinante, c'est-à-dire produite par expression dans un hôte cellulaire. L'emploi d'une collagénase recombinante offre des avantages par rapports aux collagénases naturelles (absence de contaminants, efficacité, etc.).

L'étape de dissociation est généralement réalisée à une température proche de la température du corps humain, par exemple entre 32 et 40°C, de préférence proche de 37°C, pendant une période pouvant être adaptée par l'homme du métier en fonction de la quantité d'enzyme utilisée (de 0,05 à 5 mg/ml, plus préférentiellement de 0,1 à 1 mg/ml environ). Une étape typique de dissociation selon l'invention comprend la mise en contact de l'échantillon biologique (le cas échéant traité mécaniquement comme décrit ci avant), avec la collagénase (0,5 mg/ml) pendant environ 15 heures à 37°C, sous agitation. Pour l'étape de dissociation, la collagénase peut par ailleurs être utilisée en combinaison avec d'autres enzymes, recombinantes ou d'extraction, dont la qualité aura été contrôlée.

L'invention concerne plus précisément un procédé de production de ténocytes comprenant :
- le prélèvement d'un fragment de tissu biologique comprenant des ténocytes, de préférence d'origine humaine,
- le traitement de ce fragment pour dissocier les ténocytes, en présence d'une collagénase, et
- la mise en culture des ténocytes, et leur multiplication in vitro par passages successifs, de préférence par ensemencement à une densité correspondant au quart de la confluence environ.

Les ténocytes obtenus peuvent être conservés dans différents milieux de culture (Ham, RPMI, DMEM, etc.) et/ou de conservation (solutions salines, etc.) en vue de leur utilisation extemporanée ou de leur stockage, par exemple sous forme de banques de ténocytes, comme indiqué ci-avant.

Dans ce contexte, la présente invention décrit également des compositions et méthodes particulièrement efficaces pour conserver les ténocytes, en particulier sous forme congelée. Ces méthodes permettent avantageusement de conserver les ténocytes sur de longues périodes, sans affecter leurs propriétés fonctionnelles. En outre, des compositions et méthodes particulières utilisées sont compatibles avec un usage thérapeutique, et permettent donc de préserver les ténocytes ou les compositions de ténocytes destinées à être implantées in vivo.

La congélation peut être réalisée dans différentes conditions, et en présence de différents agents ou compositions protecteurs.

Selon une première variante de réalisation, la congélation est réalisée en présence de diméthyl sulfoxyde (« DMSO »). Le DMSO est un agent protecteur bien connu, qui s'insère dans les membranes cellulaires et permet de les stabiliser, ce qui empêche la destruction des cellules. Un milieu de congélation typique au DMSO comprend par exemple de 5 à 25 % de DMSO et du sérum de veau foetal. Ce type de milieu est illustré dans les exemple et permet une conservation efficace des ténocytes, sans perte d'activité.

La congélation peut également être réalisée en absence de DMSO, notamment dans un milieu comprenant de la sérum albumine (humaine), une solution saline, et une gélatine modifiée. Un telle composition a été décrite dans la demande FR 2,746,109. Dans un autre mode préféré de mise en oeuvre, la congélation est réalisée dans un milieu comprenant de l'albumine (humaine), un polysaccharide, et éventuellement une solution saline.

Un objet particulier de l'invention réside donc dans une composition comprenant des ténocytes, notamment des ténocytes humains, sous forme congelée. L'invention concerne également des milieux pour la conservation de ténocytes, notamment de ténocytes humains, comprenant des ténocytes, notamment humains et :
- du DMSO, ou
- une gélatine modifiée, ou
- un polysaccharide, ou
- du glycérol.

L'homme du métier peut par ailleurs utiliser le procédé mentionné ci-après pour adapter la composition des milieux de congélation, notamment identifier d'autres composés utilisables pour la conservation des ténocytes sous forme congelée.

Il s'agit de préférence d'un milieu comprenant des ténocytes, une solution saline, de l'albumine humaine et une gélatine modifiée ou un polysaccharide, de préférence un polysaccharide. Encore plus préférentiellement, de telles compositions selon l'invention comprennent au moins environ 5.10⁵ ténocytes/ml.

Le milieu de conservation de l'invention est généralement préparé en mélangeant les différents constituants entre eux, puis en ajoutant ledit milieu aux ténocytes. Comme indiqué ci-avant, les ténocytes peuvent être des ténocytes primaires, fraichement isolés à partir d'un échantillon biologique, ou bien des ténocytes multipliés in vitro (par exemple par cultures monocouches).

Pour la congélation des ténocytes, la solution saline utilisée peut être plus particulièrement une solution isotonique avec le plasma. Les sels entrant dans la composition de cette solution peuvent varier. Avantageusement elle comprend des chlorures, tels que du chlorure de sodium, du chlorure de potassium, du chlorure de calcium et/ou du chlorure de magnésium, et des lactates, tels que par exemple du lactate de sodium. Dans un exemple typique, la solution saline isotonique comprend du chlorure de sodium, du chlorure de potassium, du chlorure de magnésium et du lactate de sodium. Selon une autre variante, le chlorure de magnésium est remplacé par du chlorure de calcium. Dans ce cas les concentrations en sels de la solution saline sont équivalentes ou quasi équivalentes à celles d'une solution « Ringer-lactate ». Une telle solution est habituellement utilisée en perfusion pour compenser une déshydratation ou une perte de liquide physiologique par exemple.

Selon un mode particulier de réalisation de l'invention, la solution saline est composée essentiellement de NaCI MgCl2, KCI et lactate dans des gammes de concentrations comprises respectivement entre 2 et 9 g/l ; 0,05 et 0,2 g/l ; 0,05 et 0,5 g/l et 0,5 à 5 g/l.

Les dérivés de gélatine utilisés pour la conservation des ténocytes sont plus particulièrement des gélatines fluides modifiées. De telles gélatines fluides modifiées selon l'invention sont typiquement constituées de produits d'hydrolyse du collagène modifiés chimiquement, compatibles avec une utilisation pharmaceutique. Il s'agit préférentiellement de produits ayant un poids moléculaire moyen compris entre 10 kD et 100 kD, et encore plus préférentiellement entre 15 kD et 40 kD. Ils sont préférentiellement modifiés par réaction avec un anhydride, de manière à obtenir un produit final ayant une fluidité adaptée a l'usage recherché, selon les enseignements par exemple du brevet FR 1,291,502. Il s'agit préférentiellement de l'anhydride succinique, citraconique, itaconique, aconitique ou maléique. Une gélatine fluide modifiée particulièrement avantageuse est constituée du produit d'hydrolyse du collagène ayant un poids moléculaire moyen compris entre 15 kD et 40 kD, modifié par réaction avec l'anhydride succinique. Les gélatines fluides modifiées selon l'invention peuvent être préparées par les techniques de l'homme de l'art. Parmi les gélatines fluides modifiées, on peut citer à titre d'exemple l'oxypolygélatine, obtenue par polymérisation de la gélatine avec le glyoxal et oxydation par H₂O₂. D'autres gélatines fluides modifiées sont obtenues par réaction de la gélatine (ayant de préférence une gamme de poids moléculaire d'environ 15.000 à 36.000) avec l'anhydride succinique, citraconique, itaconique, aconitique ou maléique ou le chlorure de succinyle ou de fumaryle, comme décrit dans le brevet français n° FR1,291,502. Tous ces dérivés de gélatine sont compatibles avec une utilisation pharmaceutique et peuvent être introduits dans le courant sanguin directement en solution saline isotonique. Des gélatines fluides modifiées ont également été décrites dans les brevets US2,525,753, US2,827,419, US3,108,995.

La sérum albumine utilisée est une sérum albumine humaine (SAH), d'extraction ou recombinante. La SAH naturelle d'extraction peut être produite par purification à partir de matériel biologique d'origine humaine, par les techniques classiques de fractionnement du plasma provenant de dons de sang (Cohn et al., J. Am. Chem. Soc. 68 (1946) 459 pp), ou par extraction à partir du placenta humain, selon la technique décrite par J. Liautaud et al. (13ème Congrès International d'ABS, Budapest; A: "Purification of proteins. Development of biological standard", Karger (ed.), Bale, 27 (1973) 107 pp). De préférence l'albumine purifiée utilisée dans le cadre de la présente invention est une albumine plasmatique. Tout particulièrement on peut utiliser une solution d'albumine plasmatique commerciale. La SAH recombinante peut être fabriquée dans différents types d'hôtes cellulaires, de préférence eucaryote (Cf FR 2,746,109).

Dans les milieux de conservation, le polysaccharide utilisé peut être de structure et de poids moléculaire variable. Préférentiellement, il s'agit d'un polysaccharide sulfaté, ayant de préférence un poids moléculaire compris entre 5000 et 500 000 dalton, plus préférentiellement entre 30 000 et 250 000 daltons. Le polysaccharide peut être choisi par exemple parmi le dextran (40 000 ou 60 000 daltons), l'amidon, l'hydroxyéthylamidon (240 000 dalton), etc.

Dans un mode particulier de mise en oeuvre, le milieu de l'invention est composé de plasmion (FR 2,042,381) auquel de la sérum albumine humaine est ajoutée, à des concentrations variables.

Dans un autre mode, préféré, de mise en oeuvre, le milieu de l'invention est composé de Rhéomacrodex^{R}, Hémodex^{R}, Plasmaclair^{R} ou Hestéril^{R}, auquel de la sérum albumine humaine est ajoutée, à des concentrations variables (de 5 à 45% pour une solution d'albumine à 20%).

Si nécessaire, les concentrations respectives des différents constituants peuvent être ajustées en utilisant la méthodologie suivante :

Sur des plaques multi-puits, sont répartis, dans chaque puits, chacun des constituants du milieu à une concentration fixe, à l'exception d'un constituant dont on fait varier la concentration. Le cas échéant, plusieurs plaques sont préparées, permettant de tester simultanément des conditions de concentrations différentes pour chacun des constituants, ou, si le nombre de puits est suffisant, ces différentes conditions sont testées sur une même plaque. Préférentiellement, chaque condition est testée au moins en double, de préférence en triple sur la plaque. Une préparation de ténocytes est alors introduite dans chaque puits, à une concentration de l'ordre de 10⁶ cellules /ml. La plaque est placée dans l'azote liquide à -80°C (éventuellement après une étape intermédiaire au congélateur). Les plaques congelées sont ensuite décongelées, et la viabilité cellulaire est déterminée dans chaque puits, par exemple par coloration au bleu trypan. Chaque plaque peut être lue au moyen d'un système robotisé, de manière à permettre de traiter de nombreuses conditions et d'analyser aisément les résultats obtenus.

Les compositions de ténocytes selon l'invention peuvent être conservées sous forme congelée, par exemple à -80°C, sans affecter significativement les propriétés fonctionnelles des cellules. Comme indiqué dans les exemples, une viabilité cellulaire supérieure à 85% peut être obtenue.

La présente invention montre par ailleurs que les ténocytes peuvent être modifiés génétiquement, in vitro, ex vivo ou in vivo, pour contenir un acide nucléique recombinant. Cet aspect de la présente invention permet ainsi de conférer aux ténocytes, in vitro, ex vivo ou in vivo, des propriétés biologiques et des fonctionnalités particulières, améliorant par exemple le pouvoir thérapeutique de ces cellules.

Un autre objet de l'invention réside donc dans un ténocyte humain modifié génétiquement, c'est-à-dire contenant un acide nucléique recombinant.

Un autre objet particulier de l'invention réside dans un ténocyte équin modifié génétiquement, c'est-à-dire contenant un acide nucléique recombinant.

La présente invention concerne également tout ténocyte modifié génétiquement (i.e., d'origine humaine ou animale, par exemple équine), caractérisé en ce qu'il contient un acide nucléique recombinant codant un facteur de croissance ou un facteur inhibant la réponse inflammatoire. De tels ténocytes présentent des propriétés avantageuses de reconstitution de tissu biologique. L'acide nucléique peut avoir été introduit dans la cellule elle-même ou dans une cellule parente de celle-ci, par différentes techniques de transfert de gène (vecteur viral, non viral, ADN nu, bombardement, méthode électrique, etc).

A cet égard, la présente invention décrit également des méthodes particulièrement efficaces pour le transfert de gènes dans les ténocytes in vitro, ex vivo ou in vivo. Ces techniques reposent notamment sur l'emploi d'ADN nu, d'ADN compexé à un agent facilitant (par exemple un polymère cationique) ou encore d'un rétrovirus recombinant de type GALV ou pseudotypé avec une enveloppe GALV.

L'invention concerne notamment toute méthode non-virale de transfert d'un acide nucléique recombinant dans un ténocyte, humain ou animal, comprenant la mise en contact d'un ténocyte, in vitro, ex vivo ou in vivo, avec l'acide nucléique recombinant. Ces méthodes non-virales selon l'invention comprennent par exemple :
- la mise en contact directe sous forme d'ADN nu
- la mise en contact en présence d'agent(s) facilitant la transfection, par exemple de polymères cationiques (Polyéthylèneimine), de lipides cationiques ou de peptides,
- le bombardement ou encore
- l'application d'un champs électrique.

Une méthode particulièrement préférée repose sur l'utilisation d'ADN nu. Ainsi, un objet particulier de l'invention réside dans une méthode de transfert de gènes dans un ténocyte comprenant la mise en contact d'un ténocyte, in vitro, ex vivo ou in vivo avec un acide nucléique recombinant nu.

L'invention réside encore dans l'utilisation :
- d'une composition comprenant de l'ADN nu, pour la préparation d'une composition destinée au transfert de cet ADN dans les ténocytes in vivo ;
- d'une composition comprenant un rétrovirus recombinant pseudotypé avec une enveloppe GALV, pour la préparation d'une composition destinée au transfert de cet ADN dans les ténocytes in vivo.
- d'un acide nucléique recombinant pour la préparation d'une composition destinée à la modification génétique in vivo d'un ténocyte humain.

La présente invention concerne également l'utilisation des ténocytes pour le traitement de différentes pathologies ou déficiences, aussi bien chez l'homme que chez l'animal (notamment les chevaux).

Ainsi, un autre objet de l'invention réside dans l'utilisation de ténocytes pour la préparation d'une composition destinée à l'implantation in vivo.

L'invention concerne plus particulièrement l'utilisation de ténocytes pour la préparation d'une composition destinée à la mise en oeuvre d'une méthode de traitement thérapeutique, diagnostique ou chirurgical du corps humain ou animal. S'agissant des animaux, on cite plus particulièrement les chevaux, notamment les chevaux de course, qui sont sujets à différentes altérations des tendons ou ligaments, de type tendinite. Ces chevaux sont soit abattus, soit traités par peignage tendineux, ce qui génère cependant des tendons plus rigides ou épais, dont les propriétés mécaniques ne sont pas bonnes. L'invention permet par des implantations de ténocytes autologues (ou allogéniques ou xénogéniques), par exemple à partir de banques préétablies, de reconstituer des tendons ayant des propriétés mécaniques similaires aux tendons non altérés.

Les ténocytes utilisés dans ces méthodes de traitement sont préférentiellement autologues, c'est-à-dire obtenus à partir du sujet auquel ils sont réimplantés. Comme indiqué ci-avant, il s'agit généralement de ténocytes multipliés in vitro, éventuellement modifiés génétiquement, par exemple pour coder un facteur de croissance. Il peut toutefois s'agir également de ténocytes allogéniques (i.e. provenant d'un autre sujet de la même espèce) ou xénogéniques (i.e., d'une autre espèce).

L'invention décrit également des méthodes de restauration de défauts de tissus in vivo, comprenant l'administration, à un sujet, de ténocytes, de préférence autologues, par exemple multipliées in vitro. Les cellules peuvent en outre être modifiées génétiquement. L'administration peut être réalisée de différentes manières. Il s'agit préférentiellement d'une injection péritendineuse (dans la gaine entourant le tendon) ou intratendineuse (à l'intérieur même du tendon, par exemple entre les fibres). L'injection peut être réalisée par voie percutanée, lors d'actes de microdissection permettant par exemple de dilacérer (écarter) le tendon, notamment au cours d'un peignage.

Préférentiellement, les doses de cellules injectées sont comprises entre 10⁴ et 10⁹, plus préférentiellement entre 5.10⁵ et 5.10⁷ cellules par injection. Une injection typique comprend 10⁶ à 10⁷ cellules. Par ailleurs, deux injections successives peuvent être réalisées (voire plus), si nécessaire. La quantité précise et le nombre d'injections peuvent être adaptés par l'homme du métier en fonction du sujet, de la sévérité, localisation et/ou du volume du défaut à traiter, de la présence de cellules modifiées génétiquement, etc. En particulier, dans le cadre de la régénération d'un défaut musculaire (par exemple un déchirement musculaire), des quantités plus importantes de cellules peuvent être administrées.

L'utilisation de ténocytes selon l'invention est particulièrement avantageuse, en chirurgie orthopédique ou pour la réparation des lésions musculaires. En effet, les ténocytes possèdent une activité métabolique de régénération tissulaire importante, adaptée aux nécessités locales de réparation. L'emploi de cellules issues de structures anatomiques proches des structures à réparer constitue un autre avantage majeur, dans la reconstitution de tissus ayant de bonnes propriétés mécaniques. L'emploi de ténocytes selon l'invention devrait ainsi permettre de diminuer le délai de cicatrisation spontanée des structures fibreuses (tendons, ligaments, etc.) qui est souvent long (supérieur à 45 jours) et incomplet. Dans ce but, on utilise préférentiellement, pour la réparation de lésions des tendons, des compositions de ténocytes provenant (ou dérivés) de tendons, et pour la réparation de lésions des ligaments, des compositions de ténocytes provenant (ou dérivés) de ligaments. L'utilisation des ténocytes, multipliés in vitro, permet non seulement d'augmenter la composante cellulaire du tissu lésé, mais également de servir de vecteur pour l'expression d'acides nucléiques recombinants. A cet égard, un autre avantage de l'invention réside dans l'absence de migration, hors de la structure anatomique traitée, des ténocytes. Ceci permet notamment un effet localisé, en particulier lors du transfert de gènes ex vivo ou in vivo. En outre, le fait que la délivrance du produit biologique recombinant s'effectue au contact même de la cellule effectrice favorise l'intensité de la réponse. Enfin, le chemotactisme existant lors de processus cicatriciels favorise également l'attraction des ténocytes transduits et/ou injectés au sein de la zone lésionnelle, augmentant encore l'effet local.

Une autre application des techniques d'expansion cellulaire in vitro selon l'invention concerne la réparation de lésions musculaires traumatiques. Ainsi, dans les ruptures plus ou moins étendues, le traitement conservateur est la règle, mais au prix d'une cicatrisation lente. En outre, on observe parfois la persistance d'une encoche musculaire accompagnée d'une perte de puissance musculaire et d'une risque de comblement de la zone rompue par un tissu cicatriciel fibreux diminuant les qualités mécaniques du muscle réparé. L'injection (par exemple percutanée) d'une composition contenant des ténocytes (ou des myoblastes) selon l'invention permet un comblement rapide de la zone rompue, tout en conservant les qualités mécaniques du muscle.

L'invention concerne donc également des compositions et méthodes pour :
- reconstituer la composante cellulaire de tendons, ligaments ou muscles,
- faciliter la cicatrisation des ligaments, tendons ou muscles,
- traiter des défauts de tissus biologiques, comme par exemple des défauts de tendons, ligaments, muscles squelettiques, etc, ou encore
- produire in vivo des facteurs biologiques, par administration de ténocytes modifiés génétiquement ex vivo ou par modification génétique des ténocytes in vivo.

Dans un mode particulier de l'invention, l'acide nucléique recombinant code un facteur de croissance et l'invention permet de produire in vivo un facteur de croissance de la composante cellulaire du tendon ou bien de sa vascularisation, dans le but d'améliorer la régénération tendineuse ou ligamentaire ou, plus généralement de tissus biologiques conjonctif et/ou fibreux ou musculaire, tels le muscle squelettique par exemple), et/ou de produire un ou plusieurs facteurs réduisant les réponses inflammatoires.

On entend par facteur de croissance toute protéine, polypeptide ou peptide susceptible de provoquer des réponses biologiques spécifiques comme, la chémotaxie, la prolifération cellulaire, la synthèse des fibres collagène et des protéines de la matrice, l'induction de la néovascularisation et la sécrétion d'autres facteurs de croissance.

Parmi les facteurs de croissance utilisables dans le cadre de la présente invention, on peut citer notamment :
- Le TGF-β (Transforming Growth Factor β), qui semble un médiateur impliqué dans la cicatrisation du tendon car sa présence est corrélée aux phénomènes de réparation du tendon (5),
- Le bFGF (basic Fibroblast Growth Factor), qui joue un rôle important dans la prolifération des ténocytes et dans l'angiogenèse (6). En effet, ses récepteurs sont exprimés pendant toute la période de néo-vascularisation. Le bFGF est produit par les cellules inflammatoires, mais aussi, par les ténocytes. Cette production est régulée par l'environnement cicatriciel.
- Le EGF (Epidermal Growth Factor), qui stimule la prolifération des ténocytes et dont les récepteurs sont présents pendant une grande partie du processus cicatriciel. L'expression constante de récepteurs au bFGF et le pic d'expression pour les récepteurs à l'EGF dans la membrane synoviale, indiquent que la synoviale intervient dans la migration des ténocytes et la revascularisation d'une lésion ligamentaire. L'augmentation des récepteurs se produit également au niveau du site d'insertion du ligament, zone de vascularisation importante, permettant la migration cellulaire par un effet chémotactique.
- Le PDGF (Platelet Derived Growth Factor), qui exerce, sur les ténocytes, des activités mitogène et chémotactique. Il stimule également la synthèse de collagène (7).
- le VEGF (« Vascular Endothelial cell Growth Factor ») dont l'expression permet d'augmenter la vascularisation des tendons ou ligaments ou muscles, et ainsi d'aider à la reconstitution de tissus défectueux.

La période idéale d'administration de gènes codant de tels facteurs de croissance, ou de cellules contenant de tels gènes, dans un but thérapeutique, se situe préférentiellement entre le 1^{er} et le 7^{ème} jour après la lésion, qui correspond à la période d'expression optimale des récepteurs. Il est entendu que l'administration peut être réalisée à des périodes différentes (notamment plus tardives).

Ces différents facteurs de croissance et leurs récepteurs spécifiques ont ainsi été détectés pendant les phases successives de la cicatrisation ligamentaire et tendineuse. Dans de nombreuses circonstances, la réparation tissulaire est insuffisante, soit par défaut de cicatrisation intrinsèque (ligament croisé antérieur par exemple), soit par l'existence de facteurs particuliers diminuant les aptitudes de ces tissus à cicatriser (vieillissement, prise de corticoïdes, diabète, etc.). Il a été montré, dans le ligament croisé antérieur, une présence insuffisante en médiateurs et en récepteurs spécifiques. Il est possible que cela puisse également expliquer les difficultés de réparation du tissu tendineux.

Parmi les facteurs inhibant la réaction inflammatoire utilisables dans le cadre de la présente invention, on peut citer notamment l'interleukine-10, le récepteur de l'interleukine-1, des coricomimmétique ou, plus généralement, tout popypeptide capable d'inhiber le développement d'une réponse inflammatoire.

Par ailleurs, l'acide nucléique recombinant peut également coder d'autres produits biologiques d'intérêt, comme des produits de marquage, des produits présentant une toxicité conditionnelle (de type thymidine kinase par exemple), des enzymes, des composés de la matrice extracellulaire, etc.

La présente invention permet à présent une libération locale, à des doses non-toxiques, de tels facteurs de croissance ou autres produits biologiques, et permet ainsi d'améliorer les propriétés mécaniques et structurelles des tendons et des ligaments en cours de cicatrisation. Ces différents produits biologiques peuvent être utilisés seuls ou en combinaisons. La présente invention montre à présent qu'il est possible d'implanter, dans les tissus fibreux d'un sujet (par exemple dans un tendon ou un ligament) des cellules modifiées génétiquement capables d'exprimer un polypeptide, et que ce polypeptide diffuse ou est au contact des cellules présentes dans le tendon (ou tissus fibreux).

Comme indiqué ci-avant, l'invention a donc pour objet l'utilisation de ténocytes pour la préparation d'une composition destinée à l'implantation chez l'homme, en particulier pour traiter des défauts de tissus fibreux, tels que ligaments, tendons ou muscle squelettique, par exemple.

Le terme « traiter des défauts» désigne plus particulièrement la restauration ou la compensation de défauts, c'est à dire en particulier la reconstitution, au moins partielle, de tissu dans des zones ou celui-ci est défectueux. De préférence, il s'agit d'une utilisation dans un contexte autologue, c'est-à-dire que l'échantillon biologique utilisé pour produire les ténocytes provient du sujet auquel les ténocytes produits seront administrés. Toutefois, comme indiqué ci-avant, l'utilisation de cellules allogéniques ou xénogéniques (lapin, cheval, porc, etc.) peut également être envisagée pour le traitement de pathologies humaines.

Plus généralement, l'invention concerne l'utilisation de toute cellule, autologue, allogénique ou xénogénique, pour la préparation d'une composition destinée à l'implantation ou d'administration dans un tendon ou ligament. Il s'agit plus particulièrement d'un ténocyte ou d'un fibroblaste, cultivé in vitro, éventuellement modifié génétiquement.

A cet égard, l'invention concerne également une méthode pour restaurer des tendons, ligaments ou muscles in vivo, comprenant l'administration à un sujet (de préférence humain) d'une composition de ténocytes. Il peut s'agir de ténocytes autologues, allogéniques ou xénogéniques, de préférence multipliés in vitro. Les ténocytes peuvent en outre être modifiés génétiquement. De préférence, on utilise des ténocytes autologues.

A cet égard, la méthode de l'invention comprend avantageusement le prélèvement d'un échantillon de tendon ou ligament sain chez un sujet, la préparation de ténocytes à partir de cet échantillon, puis l'administration, à ce sujet, d'une composition de ténocytes ainsi obtenus.

Selon une autre variante, l'invention concerne une méthode pour restaurer des tendons ou ligaments in vivo, comprenant l'administration à un sujet d'une composition de fibroblastes Il peut s'agir de fibroblastes autologues, allogéniques ou xénogéniques, de préférence multipliés in vitro. Les fibroblastes peuvent en outre être modifiés génétiquement, de préférence pour exprimer un facteur de croissance ou tout autre acide nucléique permettant au fibroblaste d'acquérir les propriétés biologiques adaptées à un usage selon l'invention, notamment un phénotype ou des caractéristiques de ténocyte, comme par exemple un gène codant pour un facteur augmentant la production de collagène, pour une composante de la matrice extracellulaire, pour un facteur d'ancrage, etc. De préférence, on utilise des fibroblastes autologues, par exemple cutanés.

Selon cette variante, la méthode de l'invention comprend avantageusement le prélèvement d'un échantillon de fibroblastes sains chez un sujet, leur modification génétique ex vivo, puis l'administration dans un tendon ou un ligament du même sujet, d'une composition de fibroblastes ainsi obtenus.

Selon une autre variante, l'invention concerne l'utilisation de ténocytes ou fibroblastes, autologues, allogéniques ou xénogéniques, pour favoriser la cicatrisation de déchirures ou ruptures musculaires. La rupture d'un muscle s'accompagne en effet d'une rétractation des deux parties rompues (ou déchirées) vers les insertions tendineuses. L'expérience a montré, par exemple lors de ruptures du quadriceps, que la meilleure thérapeutique était d'une part d'empêcher, voire de résorber l'hématome et, d'autre part, de favoriser une cicatrisation du muscle en extension, car toutes les tentatives de sutures de ces muscles ont toujours échoué. Une application avantageuse de l'invention réside donc dans l'injection de suspensions de ténocytes ou fibroblastes au sein de la lésion afin d'accélérer le processus de cicatrisation fibreuse et donc de diminuer le temps d'immobilisation du muscle.

Dans la présente invention, lorsque des fibroblastes allogéniques ou xénogéniques sont utilisés, ils peuvent provenir d'une banque établie.

Différentes techniques d'implantation peuvent être mises en oeuvre, selon le matériel implanté (suspension, matrice, etc.).

A cet égard, la présente invention décrit une méthode pour l'implantation de cellules in vivo, dans un tendon ou ligament, comprenant l'injection intratendineuse ou intraligamentaire de cellules.

Selon une autre variante, il s'agit d'une injection péri-tendineuse ou péri-ligamentaire, c'est-à-dire dans la gaine entourant le tendon ou le ligament. Selon une autre variante, les cellules sont placées à l'intérieur d'un dispositif capable de se positionner autour d'un tendon ou d'un ligament, pour enrober la partie défectueuse de celui-ci. Il peut s'agir de tout matériel biocompatible (éventuellement biodégradable) capable de former un manchon autour d'un tendon ou d'un ligament. Les cellules sont ainsi maintenues en contact avec le tissu et la zone à traiter.

L'invention réside également dans l'utilisation d'un film biocompatible, en particulier résorbable, pour la préparation d'une composition destinée à l'implantation de cellules, notamment de ténocytes sur un tendon ou un ligament défectueux. Il s'agit plus préférentiellement de ténocytes en solution liquide ou gélifiée, multipliés in vitro. Encore plus préférentiellement, il s'agit de ténocytes autologues.

Les ténocytes, compositions et méthodes selon l'invention, sont également utilisables pour l'étude de l'expression de gènes dans les ténocytes, leur différentiation, l'identification de gènes cibles, le screening de composés capables de moduler l'activité des ténocytes ou leur prolifération, etc.

D'autres aspects et avantages de la présente invention apparaîtront à la lecture des exemples qui suivent, qui doivent être considérés comme illustratifs et non limitatifs.

### LEGENDE DES FIGURES

Figure 1 : Représentation de la structure d'un tendon
Figure 2 : Vue schématique d'une section transversale d'un tendon
Figure 3 : Evolution dans le temps du nombre de ténocytes transduits par le gène LacZ, après infection rétrovirale.
Figure 4 : Evolution dans le temps du taux de ténocytes transduits par le gène LacZ, après infection rétrovirale (au neuvième passage).
Figure 5 : Evolution dans le temps du taux de ténocytes transduits par le gène LacZ, après infection rétrovirale (au 18^{ème} passage).
Figure 6 : Quantité de béta-galactosidase exprimée par les ténocytes transduits.
Figure 7 : Ténocytes de lapin transduits in vitro par un rétrovirus GALV-LacZ
Figure 8 : Transfection in vivo d'un tendon de souris par le plasmide CMV-LacZ (40µg) (96heures post transfert)
Figure 9 : Coloration in toto d'un tendon de souris, 48 heures après transfection in situ par le plasmide CMV-LacZ (40 µg).
Figure 10 : Résultats à 48 heures de l'implantation dans un tendon rotulien de lapin de ténocytes modifiés ex vivo par le plasmide CMV-LacZ.
Figure 11 : Résultats à 48 heures de l'implantation dans un tendon rotulien de lapin de ténocytes modifiés ex vivo par le plasmide CMV-LacZ.
Figure 12 : Courbes de croissance représentant les premier, second et troisième passages (P1, P2, P3) de cultures primaires de ténocytes préparées par dissection et digestion à la collagénase de fragments de tendon d'Achille humain issu d'un sujet de 28 ans. (12a) cultures directes ; (12b) cultures après congélation/décongélation des cellules.
Figure 13: Image en microscopie de ténocytes humains transduits par des rétrovirus codant la protéine LacZ, après coloration au X-gal.

### MATERIEL & METHODES

### 1. Vecteurs

Les vecteurs utilisés *in vitro* ont été des plasmides et des vecteurs rétroviraux.

### 1.1. Plasmides

### Plasmide avec gène codant pour la β-galactosidase

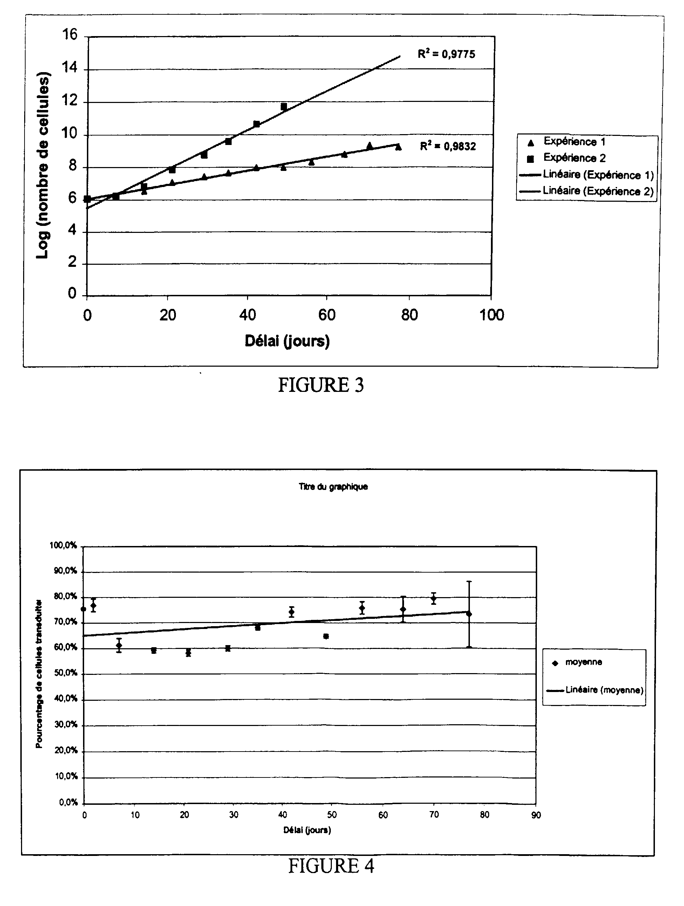

Le plasmide utilisé était le plasmide CMV-LacZ, un ADN plasmidique modifié d'Escherichia Coli (pCMVβ, produit par Clontech®). Ce plasmide possède un promoteur CMV, issu du cytomégalovirus humain, permettant l'expression forte du gène LacZ, originaire de l'Escherichia Coli, codant pour la β-galactosidase (β-Gal). La séquence est terminée par un signal de polyadénylation (poly A) provenant du virus SV 40 (simian virus). Le plasmide contient par ailleurs une origine de réplication provenant d'E.Coli (Col E1 Ori) et un gène de sélection permettant la résistance à l'ampicilline (figure 3).

Les étapes de production du plasmide CMV-LacZ comprenaient (8):
- culture sur milieu de sélection des bactéries renfermant le plasmide,
- extraction de l'ADN plasmidique par lyse alcaline,
- récupération du culot bactérien,
- élimination de l'ADN génomique bactérien,
- précipitation de l'ADN plasmidique,
- purification par double gradient de chlorure de césium (précipitation des protéines, ultracentrifugation 2 fois 6 heures à 55000 rpm à 20°C, puis dialyses),
- précipitation de l'ADN plasmidique (concentration de l'ADN).

### Plasmides avec gène codant pour la green fluorescent protein

D'autres structures plasmidiques codant pour un gène marqueur de type GFP (green fluorescent protein) ont été utilisées. Ce type de gène permet une détection par fluorescence sous l'action de rayons ultra-violets. Les plasmides utilisés étaient le SV40-GFP (promoteur du Simian Virus 40) et le PET 47. La méthode de production était identique à celle décrite précédemment.

### 1.2. Vecteurs rétroviraux

Deux types de vecteurs rétroviraux transduisant le gène nls-LacZ ont été utilisés. Ce gène permet le marquage, en bleu, du noyau des cellules transduites après coloration par le X-gal (5-bromo-4-chloro-3-indolyl-D-b-galactopyranoside). Les vecteurs rétroviraux produits dérivaient, pour les uns, du virus de la leucémie murine de Moloney (Mo-Mulv) et, pour les autres, du virus de la leucémie aiguë du Gibbon (Gibbon Ape Leukemia Virus (GALV) (9). Les lignées d'encapsidation étaient, dans le 1^{er} cas, des Ψcrip LLZ (Ψcrip-LLZ-nls LacZ) et, dans le 2^{ème} cas, des GALV 18. Ces lignées sont décrites plus loin.

Les surnageants de production des virus ont été récupérés et purifiés par un filtre de 0,45 µm (élimination des cellules d'encapsidation et des débris cellulaires), puis la qualité des stocks a été vérifiée.

Les surnageants produits ont été répartis, dans des tubes stériles étanches, sous forme d'aliquots afin d'éviter des congélations successives. Les tubes étaient alors conservés à -80°C.

### 2. Cultures cellulaires

### 2.1. Ténocytes

Des ténocytes ont été isolés à partir de tendons d'Achille de souris mâles adultes de type OF1, SPF (specific pathogen free), d'environ 40 g (IFFA-CREDO®), ou de tendons rotuliens de lapins mâles de type New Zealand, SPF (specific pathogen free), d'environ 2,5 Kg (ESD® -France). Les ténocytes humains sont obtenus dans des conditions similaires.

### 2.2. Lignées d'encapsidation

Deux types de lignée d'encapsidation ont été utilisés pour la production des vecteurs rétroviraux :

La lignée GALV 18, produite par F.L. Cosset, qui produit des rétrovirus recombinants dont l'enveloppe est celle du virus GALV responsable de la leucémie aiguë du Gibbon. Ces cellules d'expression dérivent de cellules d'ostéosarcome de type TE 671 qui ont été transfectées par des vecteurs apportant les gènes gag-pol, d'une part, et env, d'autre part, pour aboutir, après sélection, aux cellules TE FLY GA délivrant des particules virales vides. Après transfection par un vecteur contenant le gène nls-LacZ, les cellules d'encapsidation GALV 18 ont été obtenues.

Les cellules d'encapsidation de type ΨcripLLZ (Ψcrip-LLZ-nls LacZ) sont des fibroblastes de souris producteurs de particules virales recombinantes amphotropes, dont l'enveloppe est celle du virus de la leucémie murine de Moloney.

### 2.3. Milieux de culture cellulaire

### Milieu de base :

Deux types ont été utilisés, le DMEM (milieu de Eagle modifié par Dulbecco, référence 41966, Life Technologies®) et le HAM F12 (milieu de Ham, référence 21765, Life Technologies®) (10,11).

### Supplémentation :

Elle nécessitait:
- du sérum de veau foetal (FCS, fetal calf serum, laboratoire HyClone®), inactivé par la chaleur, à 10 p.100 du volume ou du sérum de veau nouveau-né (NN, laboratoire HyClone®),
- de la L-Glutamine à 4 mM/I (Gibco®),
- un mélange d'antibiotiques PSN (pénicilline, streptomycine, néomycine) à 1X (stock 100X, référence 15640-022, Life Technologies®),
- de la vitamine C (acide ascorbique ) à 50 mg/I (Sigma®, réf : 44-03) dans certains cas (12).

Les ténocytes de lapin ou humains ont été cultivés dans les deux types de milieu de façon comparative, supplémentés par du FCS, avec et sans vitamine C. Les ténocytes de souris ont été cultivés dans du DMEM supplémenté par du FCS et de la vitamine C. Lors des cultures, les cellules étaient ensemencées sur des supports de culture en polystyrène (Costar®) dont la taille variait de 75 à 225 cm². Ces supports étaient ensuite placés dans un incubateur à 37°C dans une atmosphère enrichie en CO₂ (5% de CO₂). Les milieux étaient systématiquement renouvelés tous les 2 à 3 jours. A confluence, les cellules étaient décollées en utilisant de la trypsine-EDTA (Life Technologies®). Un échantillon était alors analysé, après coloration au bleu trypan, afin de déterminer la concentration cellulaire, permettant de calculer le nombre total de cellules, et d'apprécier le taux de mortalité cellulaire. Le taux de réensemencement des cellules était un adapté au type de manipulation. Plusieurs lignées primaires de ténocytes (primocultures) ont été constituées à partir de tissus tendineux d'origine animale.

### 3. Analyse des transferts de gènes in vivo

### 3.1. Technique de coloration sur lame

Dès le prélèvement sur les animaux, les tissus étaient plongés dans l'azote liquide puis transportés au laboratoire d'histologie. Les tissus, après inclusion dans une résine, étaient découpés au microtome, en coupes de 5 à10 µm d'épaisseur. Les coupes de tissu étaient alors déposées sur lames. La fixation des coupes, d'une durée de 30 min, utilisait une solution de PBS contenant 1% de formaldéhyde et 0,2% de glutaraldéhyde. Après 2 rinçages au PBS, les coupes étaient laissées dans la solution de X-Gal pendant 4 heures. La lecture des coupes était améliorée par la contre-coloration de quelques coupes par l'HE (hématéine-éosine) soulignant les noyaux cellulaires et permettant d'identifier la présence d'une infiltration de cellules mononuclées.

### 3.2. Technique de coloration in toto

Cette technique permettait de détecter une activité β-galactosidase par la présence, dans le tissu monobloc étudié, d'une coloration bleutée visible macroscopiquement.

Après prélèvement sur les animaux, les tissus étaient lavés 2 fois dans du PBS. Pour la fixation, les prélèvements« monobloc » étaient plongés, pendant 30 min, dans une solution de PBS contenant 1% de formaldéhyde et 0,2% de glutaraldéhyde. Après 2 rinçages au PBS, les tissus étaient laissés dans la solution de X-Gal durant toute la nuit (O.N.), à une température de 32°C. Après 2 rinçages au PBS, les tissus étaient inclus dans la paraffine. Des coupes, de 5 à10 µm d'épaisseur, centrées sur les zones bleues étaient réalisées puis déposées sur lames. La contre-coloration de quelques coupes par l'HE permettait de souligner les noyaux cellulaires.

### 3.3. Analyse macroscopique

L'examen du tendon était réalisé lors du prélèvement mais aussi après coloration de la pièce *in toto.*

Lors du prélèvement, l'aspect global du tendon et des tissus péritendineux était étudié, ainsi que l'aspect de l'articulation de voisinage (inspection du cartilage, des ménisques et de la membrane synoviale). Les signes d'inflammation, une modification de volume ou de texture du tendon, et l'existence d'adhérences étaient particulièrement recherchés.

Après coloration de la pièce *in toto,* une analyse macroscopique du prélèvement permettait la recherche d'une coloration bleutée, traduisant la présence de cellules transduites. Cette recherche était effectuée, dans et autour du tendon, en précisant la taille, la situation et la diffusion de cette coloration.

### 3.4. Analyse microscopique

Les inclusions de tissus, les coupes et les interprétations de lames ont été réalisées. Les techniques employées étaient, soit la technique de coloration sur lame (technique classique), soit la technique de coloration *in toto.*

L'analyse des lames s'attachait à mettre en évidence la présence d'une coloration bleue signant la présence de cellules transduites dans le tendon. Le bleu était strictement nucléaire avec le gène nls-LacZ, et, nucléaire et cytoplasmique avec le gène LacZ. La distribution des zones bleutées était notée. Par ailleurs, l'infiltration de cellules mononucléées ou un aspect d'hypervascularisation, traduisant une réaction inflammatoire, étaient recherchés autour des zones bleutées. La lecture était améliorée par la contre-coloration à l'HE. L'étude de tendons injectés, dans les mêmes conditions sans gène LacZ, permettait de vérifier l'absence d'activité β-Galactosidase endogène.

### EXEMPLE 1 : PRODUCTION ET CULTURE DE TENOCYTES

Cet exemple décrit la production et la culture de ténocytes obtenus à partir de fragments de tissus sains.

Après prélèvement du tendon, réalisé sur le sujet dans des conditions d'asepsie, les cellules ont été obtenues par deux étapes :
- le découpage mécanique du prélèvement tendineux en fragments millimétriques, à l'aide de ciseaux fins. Les fragments étaient ensuite mis en culture.
- la digestion enzymatique par la collagénase de type D à faible activité trypsinique (Boehringer®,réf. 1.088.866). Une phase de digestion initiale utilisait une solution de HBSS (Solution saline équilibrée de Hanks, Life Technologies®) renfermant 1 mg/ml de collagénase (soit 112 U/ml) sous un volume équivalent à 5 ml de solution pour 0,1 g de tissu à digérer (10). Le tube était placé, dans l'incubateur à 37°C, sur un agitateur rotatif, pendant une heure. Une seconde phase de digestion utilisait une solution renfermant 0,25 mg/ml de collagénase sous un même volume. Le tube était alors placé dans l'incubateur à 37°C O.N. (over-night). Le lendemain, le contenu du tube était passé sur un filtre de 70 µm (cell strainer®) puis mis en culture dans une flasque de 25 cm² après 2 lavages au PBS (Dulbecco's phosphate buffered saline, Gibco®).

Les cultures primaires étaient réalisées dans les conditions décrites plus haut. Le milieu était renouvelé toutes les 48 H, le repiquage effectué lorsque les cellules arrivaient à confluence.

Lors de deux manipulations distinctes réalisées sur deux lapins différents, l'apparition des premières lignées cellulaires était observée à partir du 13ème jour après la dilacération mécanique du prélèvement tendineux (1/3 du tendon rotulien, soit environ 160 mg). Lors de l'association à la digestion enzymatique par la collagénase D, dans deux autres manipulations, les premières lignées cellulaires apparaissaient vers le 4ème jour. La confluence, sur un support de 25 cm², était obtenue aux alentours du 21^{ème} jour après dilacération mécanique isolée et aux alentours du 16^{ème} jour après dilacération mécanique associée à la digestion enzymatique O.N.

Lors de deux manipulations distinctes, la dilacération mécanique d'un tendon d'Achille de souris, associée à la digestion enzymatique par la collagénase D, a permis d'isoler le 1^{er} jour de 5 à 10³ cellules par tendon (d'environ 25 mg). La confluence, sur un support de 25 cm², était obtenue en 12 jours et un nombre de cellules, de l'ordre de 3.10⁶ cellules, était obtenu en 21 jours.

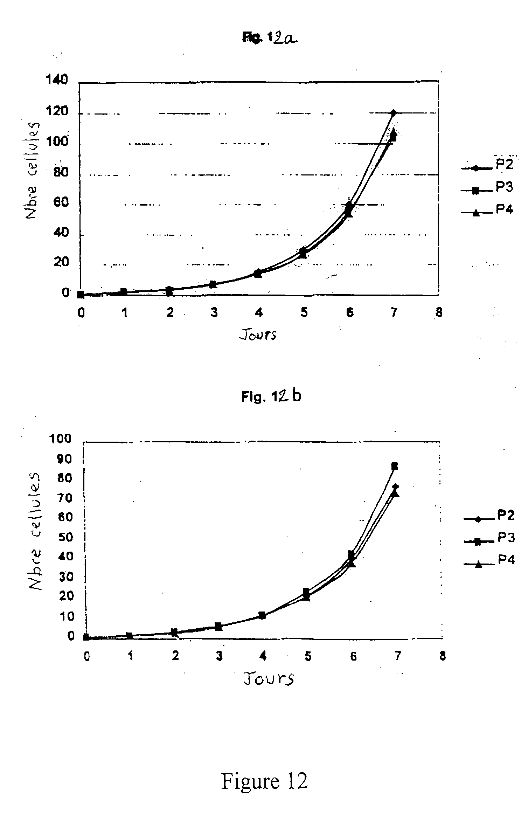

Lors de plusieurs manipulations distinctes, la dilacération mécanique de fragments de tendon d'Achille humains, associée à la digestion enzymatique par la collagénase, a permis d'isoler des ténocytes humains, et de placer et maintenir ceux-ci en culture. Les résultats présentés sur la Figure 12a montrent que les ténocytes humains, aux différents passages, présentent une capacité de prolifération (expansion) in vitro importante, permettant de générer suffisamment de cellules viables et biologiquement fonctionnelles pour des applications thérapeutiques.

Les ténocytes isolés à partir de tendons de lapins NZ étaient de grosses cellules fusiformes d'allure fibroblastique.

A confluence, la densité cellulaire était d'environ 2,8.10⁴ cellules par cm². Cette valeur a été constatée sur différentes tailles de support de culture cellulaire (de 25 à 220 cm²) en laissant les cultures approcher de la confluence maximale en monocouche. Au-delà de la confluence, ces cellules se développaient sur des couches superposées et cela ne permettait plus de les séparer correctement. La taille des ténocytes a été estimée par le rapport de la valeur de la surface du support de culture sur le nombre de ténocytes à confluence. Cette taille était de l'ordre de 3,6.10³ µ². Compte tenu des observations microscopiques qui montraient que le rapport de longueur sur largeur était d'environ 4/1, la taille des ténocytes était estimée à environ 120 µ de long sur 30µ de large. Cependant, il a été observé que la taille et la forme de ces cellules variaient selon le degré de confluence et la fréquence de repiquage.
Des essais de culture primaire utilisant des taux d'ensemencement variables (du 1/16 de confluence à ¾ de confluence) ont montré que le développement des ténocytes semblait optimal lors de l'ensemencement au ¼ de confluence. Cela a été évalué de façon semi-quantitative en appréciant le temps de doublement et en tenant compte de la fréquence de repiquage. En effet, les décollements devaient être réguliers avec une fréquence inférieure à 8 jours. Au-delà de ce délai, même s'ils n'étaient pas à confluence, les ténocytes nécessitaient un temps de contact prolongé avec la trypsine, le taux de mortalité cellulaire devenait important et la dissociation des ténocytes était de mauvaise qualité.

Le temps de doublement cellulaire a été estimé sur deux cultures de ténocytes, issus d'un même lapin, poursuivies au-delà de 45 jours. Les conditions de cultures étaient identiques dans les deux expériences. Les ténocytes étaient ensemencés, au ¼ de confluence (7.10³ cellules / cm²), sur un support de 150 cm², le milieu était renouvelé tous les 2 jours et le repiquage effectué une fois par semaine.
Les ténocytes de lapin étaient au 9^{ème} passage dans l'expérience 1 et au 18^{ème} passage dans l'expérience 2. Le temps de doublement des ténocytes variait, de façon importante, dans une même culture dans des conditions de repiquages réguliers. La vitesse de croissance des ténocytes, par diminution du temps de doublement, tendait à augmenter avec le nombre de passages. Les résultats sont rapportés dans le tableau I.

Des ténocytes humains sont obtenus dans les mêmes conditions, à partir de tendons ou fragments de tendons humains, tels que tendons de la patte d'oie, tendon rotulien ou tendon du muscle petit palmaire.

### EXEMPLE 2 : CONGELATION DES TENOCYTES

Des méthodes de conservation par congélation des ténocytes ont été développées afin de pouvoir facilement disposer de cellules pour les différentes expérimentations, sans avoir besoin de recourir à de nouvelles extractions.

Cet exemple montre que les ténocytes primaires ou secondaires peuvent être congelés sans altérer leur viabilité.

Les congélations ont été réalisées en présence de DMSO, dans un milieu 1/2 volume de sérum de veau nouveau-né et 1/2 volume d'un mélange de RPMI renfermant 20% de DMSO. Les tubes étaient conservés à -80°C puis dans l'azote liquide. Des résultats positifs peuvent également être obtenus en réalisant la congélation des ténocytes en présence de milieux dépourvus de DMSO et/ou de glycérol, tels que décrits dans la description.

Toutes les expériences de congélation-décongélation (environ 30 décongélations) ont montré la bonne tolérance des ténocytes à la technique de congélation utilisée. Après décongélation, le taux de mortalité était faible et les cellules retrouvaient des caractéristiques de croissance *in vitro* similaires aux cellules de passage identique et non congelées. Ces résultats sont d'autant plus intéressants que les ténocytes sont des cellules à vie limitée dont le nombre de divisions est restreint (13). En définitive, dans des délais relativement courts, il est possible d'obtenir un nombre élevé de cellules que l'on peut congeler en vue d'une utilisation ultérieure. A cet égard, la Figure 12b démontre les capacités de survie et de croissance de ténocytes humains après congélation/décongélation. Cette figure montre ainsi que les ténocytes humains peuvent être congelés et décongelés, que ces cellules restent viables et cultivables, qu'elles peuvent être multipliées in vitro après décongélation, et restent biologiquement fonctionnelles.

### EXEMPLE 3 : TRANSFERT DE GENES DANS LES TENOCYTES IN VITRO

Cet exemple montre la, possibilité de transférer efficacement des gènes dans des ténocytes in vitro. Pour le transfert de gènes in vitro (ou ex vivo), différentes techniques peuvent être utilisées. Dans les exemples, le transfert de gènes a été étudié *in vitro* sur des ténocytes par l'intermédiaire de plasmides ou de rétrovirus.

### 3.1. Transfert de plasmides

Les plasmides testés étaient de type LacZ (CMV-LacZ) et de type GFP (SV40-GFP et PET 47). Les transfections ont été réalisées avec 10 µg d'ADN par puits de 10 cm² contenant 2.10⁵ ténocytes. Les plasmides ont été utilisés nus ou enrobés de PEI (30 µl à 10⁻² M par puits). L'efficacité du transfert était appréciée par lecture directe des puits, par comptage des cellules transduites après décollement (après coloration au X-Gal ou comptage par FACS) ou par la technique de chémi-luminescence.

Le 1^{er} jour, les ténocytes étaient mis en culture sur des supports de 9 cm² de façon à obtenir le lendemain une confluence de 60 p.100.

Le 2^{ème} jour, après retrait du milieu de culture usagé, un volume de 0,5 ml de solution de HBSS, contenant la quantité voulue de plasmide, était versé sur la monocouche de ténocytes. Puis le support était remis dans l'incubateur (à 37°C, 5% de CO₂) et un volume de 1,5 ml de milieu était ajouté 2 heures plus tard.

Le 3^{ème} jour, l'efficacité de la transfection était évaluée.
Les résultats obtenus montrent que la transfection de ténocytes in vitro par des vecteurs non-viraux est possible. En particulier, les résultats obtenus montrent un taux de transfection, calculé sur 100 cellules en présence de PEI de l'odre de 10%. L'activité β-galactosidase a été calculée, dans deux expériences, dans des puits de 10 cm² contenant des ténocytes transfectés par 10 µg d'ADN et 30 µl de PEI à 10⁻² M. Cette activité était de 9 pg et de 34,5 pg de β-galactosidase pour un témoin négatif à 0 pg. La transfection par le plasmide SV40-GFP (10 µg) enrobé de PEI (30 µl à 10⁻² M) montre également un taux de transfection calculé par FACS sur 1500 cellules de l'odre de 24%.

Ces résultats montrent donc que les ténocytes, in vitro, peuvent être transfectés par des vecteurs non-viraux, en particulier par de l'ADN plasmidique.

### 3.2. Transfert par un rétrovirus

Après leur titration sur cellules de référence, les vecteurs rétroviraux ont été utilisés pour infecter *in vitro* des ténocytes humains, de lapin et de souris. L'efficacité du transfert a été appréciée par lecture directe des puits, par comptage des cellules transduites après décollement ou par la technique de chémi-luminescence.

L'influence des conditions d'infection sur l'efficacité du transfert a été analysée en étudiant l'infection de ténocytes de lapin par des vecteurs rétroviraux de type GALV tout en faisant varier le taux de confluence des ténocytes et le rythme de renouvellement du surnageant viral.

L'efficacité de transduction des rétrovirus GALV 18 et Ψcrip-LLZ a été comparée en analysant, dans des conditions identiques, le taux et l'activité β-galactosidase des ténocytes transduits. Le taux d'expression de la β-galactosidase par un ténocyte transduit a été calculé, pour chacun des 2 types de rétrovirus, par le rapport du taux d'expression global sur le nombre total de ténocytes transduits.

Le 1^{er} jour, les cellules étaient mises en culture sur des supports de 9 cm² de façon à obtenir le lendemain la confluence souhaitée.

Le 2^{ème} jour, après retrait du milieu de culture usagé, chaque puits recevait un volume de 0,5 ml de surnageant contenant les virus, auquel était ajouté 4 µI de Polybrène (soit 8 µI / ml). Puis le support était remis dans l'incubateur (à 37°C, 5% de CO₂) et un volume de 1,5 ml de milieu frais était ajouté, dans chaque puits, 4 heures plus tard.

Le 3^{ème} jour, l'analyse de l'efficacité de transfert était effectuée.

L'appréciation de l'efficacité du transfert était, soit réalisée par lecture directe sur supports de culture (estimation semi-quantitative), soit réalisée par comptage après décollement des cellules (valeur précise du taux de cellules transduites). La fixation des cellules transduites par le gène GFP était réalisée par une solution de PBS contenant 1% de paraformaldéhyde, celle des cellules transduites par le gène Lac-Z utilisait une solution de PBS contenant 1% de formaldéhyde et 0,2% de glutaraldéhyde.

### Résultats

La titration des surnageants rétroviraux, produits par les cellules d'encapsidation Ψcrip-LLZ, a été effectuée sur des cellules 3T3 NIH (ensemencées à 2.10⁵ cellules par puits de 10cm²). Le titre était de l'ordre de 3.10⁵ pfu/ml de surnageant.

La titration des surnageants rétroviraux, produits par les cellules d'encapsidation GALV 18, a été effectuée sur des cellules HCT 116 (ensemencées à 5.10⁵ cellules par puits de 10cm²). Le titre variait de 2 à 5.10⁵ pfu/ml de surnageant.

### Infection des ténocytes de lapin par les vecteurs rétroviraux

### . Influence des conditions d'infection sur le taux de transduction virale

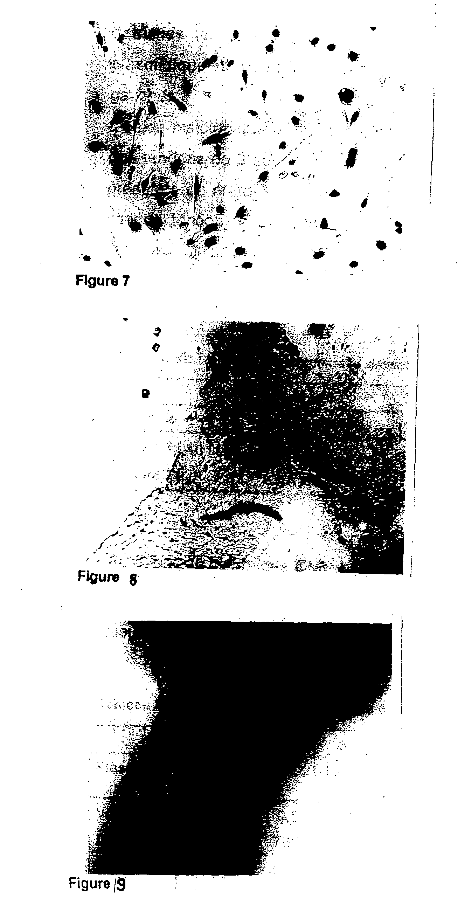

Le taux de transduction *in vitro* des ténocytes de lapin, a été mesuré en faisant varier le taux de confluence des ténocytes et les conditions de contact avec le surnageant viral GALV 18 (fig.7). Le surnageant viral était utilisé pur pendant 4 heures et était renouvelé ou non. Les résultats ont été évalués par comptage manuel du nombre de ténocytes transduits dans un échantillon de 500 cellules (tableau II). Aucun ténocyte transduit n'était présent dans le témoin non infecté.

Le taux de transduction des ténocytes a semblé optimal au ¼ de confluence et en l'absence de renouvellement du surnageant viral. Ce taux tendait à diminuer avec l'augmentation de la confluence des ténocytes et le nombre de renouvellements du surnageant.

### . Comparaison du taux de transduction par les rétrovirus GALV 18 et Ψcrip-LLZ:

La capacité d'infection des ténocytes de lapin par les vecteurs rétroviraux a été testée *in vitro* dans des puits de 10 cm², contenant 7.10⁴ cellules. Le taux de transduction des ténocytes a été calculé, à partir d'échantillons de 500 cellules, lors de 2 expériences utilisant le même surnageant viral. Le taux de transduction était de 0,6% et de 0,7% avec les rétrovirus Ψcrip-LLZ et, de 36,6% et de 39,8% avec les rétrovirus GALV 18. Dans les témoins (puits non infecté), aucun ténocyte n'était transduit.

L'activité β-galactosidase des ténocytes transduits, dans des puits de 10 cm², par ces vecteurs rétroviraux, a été mesurée dans les mêmes conditions d'expérience. Cette activité était, après infection par les rétrovirus Ψcrip-LLZ, de 106 pg et 166 pg, soit 0,25 et 0,34 pg par ténocyte transduit. Elle était, après infection par les rétrovirus GALV 18, de 6274 pg et 8876 pg, soit 0,25 et 0,32 pg par ténocyte transduit. Le bruit de fond (cellules non infectées) était de 5 pg.

### . Cinétique

L'évolution dans le temps du nombre total théorique de ténocytes transduits, après infection par des vecteurs rétroviraux de type GALV 18, est reportée dans le tableau III. Les résultats sont issus de 2 expériences utilisant des ténocytes infectés à passage 9 (exp. 1) et 18 passage (exp. 2). Compte tenu de l'évolution exponentielle, les résultats étaient exprimés en log du nombre de cellules obtenues et représentés sous forme de courbe (fig. 3). Une courbe de tendance, calculée pour chaque expérience, permettait d'apprécier la cinétique et d'extrapoler les résultats.

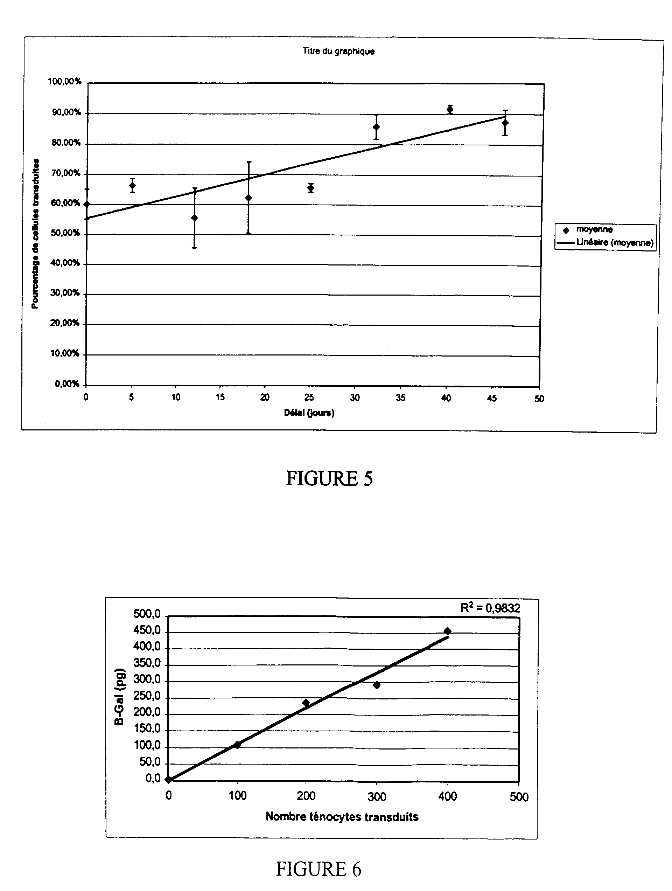

L'évolution du taux de cellules transduites a été analysée dans 2 expériences utilisant des ténocytes de lapin infectés, par des vecteurs rétroviraux de type GALV, à passage 9 (fig.4) et à passage 18 (fig.5). Les résultats, représentés sous forme de courbes, étaient exprimés par la moyenne de 2 mesures. L'intervalle de confiance avait la valeur d'un écart-type.

La présence du transgène dans les ténocytes était encore significative à 77 jours de recul de l'infection. Le taux des ténocytes transduits était relativement stable avec une tendance à l'augmentation.

### Infection des ténocytes de souris par les vecteurs rétroviraux

L'infection des ténocytes de souris, par le vecteur rétroviral Ψcrip-LLZ, a été testée *in vitro* sur des cellules au ¼ de confluence en utilisant du surnageant viral pur pendant 4 heures. Lors de l'infection de puits de 10 cm², contenant 1,4.10⁵ ténocytes, le nombre de ténocytes transduits était de 245 par ml de surnageant viral pur.

### Infection de ténocytes humains par un rétrovirus

Les ténocytes humains en culture (passage P2) ont été incubés en présence de surnageant de rétrovirus GALV18, contenant le gène nls-LacZ, dans les conditions décrites ci-avant. Trois jours après l'infection, les cellules ont été fixées à la formaldéhyde et colorées au X-gal pour révéler l'expression du gène LacZ.

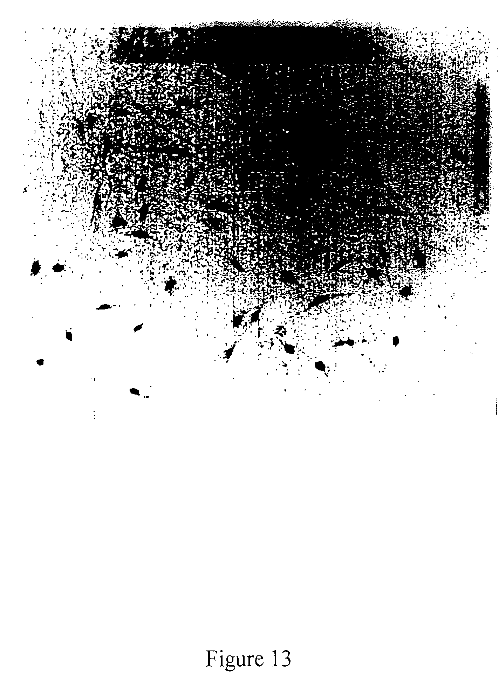

Les résultats obtenus sont présentés sur la Figure 13, et montrent une efficacité de transduction élevée des ténocytes humains. Ces résultats montrent donc que les ténocytes humains, en culture, peuvent être modifiés génétiquement de manière efficace, par l'emploi de rétrovirus, et peuvent donc acquérir des propriétés avantageuses pour les utilisations thérapeutiques de l'invention. Ces résultats sont également transposables à la modification génétique directe in vivo.

### Constitution d'une lignée transduite par le gène nls-LacZ

Des ténocytes, issus d'explants de tendons rotuliens de lapin New Zealand, ont été infectés par des vecteurs rétroviraux de type GALV 18 véhiculant le gène nls-LacZ.

Les conditions d'infection étaient l'ensemencement de ténocytes, au 1/4 de confluence, dans une flasque de 150 cm² puis leur infection, pendant 4 heures, par un surnageant viral pur et non renouvelé. Le milieu, rajouté à 4 heures, contenait du DMEM supplémenté en vitamine C.

Les cultures étaient poursuivies, dans des flasques de 150 cm², avec un repiquage au 1/4 de confluence chaque semaine. Lors de chaque décollement, l'examen d'un échantillon de cellules permettait de déterminer le nombre total de cellules obtenues. Le taux de cellules exprimant le transgène était calculé, après coloration au X-Gal, à partir de 2 mesures portant sur 500 cellules chacune.

Ces lignées, de passages 9 et 18, ont été utilisées pour analyser, après infection par des vecteurs de type GALV 18, l'évolution dans le temps du nombre des cellules transduites ainsi que la proportion de ces cellules au sein de la culture, la sélection des cellules transduites étant impossible avec le vecteur GALV 18. Les cultures étaient repiquées une fois par semaine, au ¼ de confluence, dans des supports de 150 cm². Lors du repiquage, le nombre de cellules utilisé ne tenait compte que des cellules vivantes, après coloration au bleu Trypan. Le nombre total de cellules obtenues en une semaine était mesuré après retrait des cellules mortes en culture mais tenait compte des cellules mortes lors du décollement. Le comptage manuel des cellules était effectué, par 2 observateurs indépendants, à partir d'un échantillon de la suspension de cellules. L'évolution du nombre total théorique de ténocytes a pu être calculée et représentée sous forme de courbes. Le pourcentage de cellules transduites était mesuré, après coloration au X-Gal, par comptage manuel de 2 échantillons de 500 éléments. La mesure itérative du taux de cellules transduites a permis l'étude de la stabilité de ce taux, sur une période de plus de 45 jours, à partir de 2 lignées transduites.

### EXEMPLE 4 : TRANSFERT DE GENES DANS LES TENOCYTES IN VIVO

Cet exemple montre la possibilité de transférer des acides nucléiques recombinants dans les ténocytes in vivo. En particulier, cet exemple montre la possibilité de réaliser un transfert non-viral in vivo, notamment au moyen d'ADN nu.

Dans cet exemple ,des essais de transfection par plasmide ont été effectués *in vivo* dans des tendons d'Achille.

Après détermination du volume injectable au niveau d'un tendon d'Achille de souris (< ou égal à 40 µI), des essais de transfert *in situ* ont été réalisés par l'injection de 20 et 40 µg de plasmides nus afin d'étudier la possibilité de transfection *in vivo* et d'évaluer la durée d'expression du transgène. Des expériences ont été réalisées afin d'améliorer l'efficacité du transfert. Différentes conditions de transfert ont été analysées en faisant varier la formulation du plasmide (nu ou enrobé de PEI), la quantité injectée (de 3,5 à 40 µg) et le volume injecté (de 0,5 à 40 µl). La technique d'injection était soit percutanée, soit à ciel ouvert.

Les résultats de ces transfections ont été appréciés qualitativement, par des examens macroscopiques et histologiques, et quantitativement par le dosage de l'activité β-galactosidase.

Un essai de transfert *in situ* a été réalisé par l'injection de 100 pg de plasmides nus dans des tendons d'Achille afin d'étudier la possibilité de transfection *in vivo* chez le lapin.

La tolérance des transfections a été étudiée macroscopiquement et par l'examen histologique utilisant notamment la contre-coloration des lames par l'HE.

### 4.1. Modèles animaux

Le 1^{er} modèle utilisait des souris mâles adultes de type OF1, SPF (specific pathogen free), de 40 g (IFFA-CREDO®). L'anesthésie était assurée par l'utilisation d'Avertine® (tri-bromo-éthanol, à 25 mg/ml) à la dose de 250 mg/Kg, soit environ 400 µI administré par voie IP (intra-péritonéale). Le sacrifice des souris était réalisé par dislocation cervicale.

Le 2^{ème} modèle utilisait un lapin mâle de type New Zealand, de 4 semaines, pesant 2,5 Kg (ESD 01-France). L'anesthésie était assurée par l'utilisation d'Hypnovel® 5 mg (1 ml) administré par voie IM et de Ketalar® (kétamine) à la dose de 80 mg/Kg administré par voie IM. Le sacrifice était réalisé par l'injection IV d'une dose létale (50 mg) de Nesdonal® (thiopental).

### 4.2. Injection de plasmides nus ou enrobés

Le plasmide utilisé, lors des transferts *in vivo,* était le CMV-LacZ. Un plasmide non LacZ (irelevant), par exemple un plasmide GFP, était utilisé comme témoin négatif. Dans les expériences chez les souris, les plasmides ont été utilisés à différentes concentrations (de 1 µg/µl à 7,25 µg/µl) et sous différents volumes (de 0,5 à 40 µl). Chez le lapin, 100 µg de plasmide à 1 µg/µl ont été utilisés. Les plasmides étaient utilisés seuls (plasmides nus) ou combinés au PEI.

L'injection était effectuée dans et parallèlement au tendon, de façon ascendante, soit par voie percutanée, soit après incision cutanée permettant le contrôle visuel du positionnement de l'aiguille. Des seringues délivrant des microvolumes étaient utilisées (Hamilton®). Une 1^{ère} série d'injections percutanées, avec du bleu de méthylène dilué au 1/10, a été réalisée afin de déterminer le volume maximal admissible en regard des tendons d'Achille de souris sans qu'il y ait de diffusion importante.

### 4.3. Résultats

### Transfection in vivo dans des tendons d'Achille de souris

Une première série d'injections de plasmide CMV-LacZ nu a été effectuée dans le but d'évaluer la possibilité de transfection *in situ.* L'expression du transgène a été analysée 48 heures et 96 heures après injections percutanées de plasmides CMV-LacZ nus dans les tendons d'Achille de 2 souris.

Il est apparu que le transfert après injection de 40 µg d'ADN semblait meilleur qu'après injection de 20 pg d'ADN. Le transgène était visible à J2 et à J4. Le transfert semblait juxta-tendineux et non présent sur toutes les coupes. L'examen de 2 tendons non injectés, par le plasmide CMV-LacZ, montrait l'absence d'activité β-galactosidase endogène.

La faisabilité de la transfection *in situ* au moyen du plasmide CMV-LacZ nu étant prouvée, la 2^{nde} étape a été l'étude de la durée d'expression du transgène. L'expression du gène Lac Z a été analysée à J2, J4 et J8 dans une série de 9 souris. Une injection percutanée de 40 µg de CMV-LacZ nu (1 µg/µl) était réalisée dans les tendons droits tandis qu'une injection percutanée de 40 µg d'ADN plasmidique non LacZ (irelevant) nu (1 µg/µl) était réalisée dans les tendons gauches.

L'étude histologique a été réalisée aux 3 délais par coloration au X-Gal sur lame des tendons de 3 souris. L'examen de coupes transversales étagées montrait la présence du marqueur dans les tendons droits (sur une partie des coupes de chaque tendon) et l'absence du marqueur sur toutes les coupes de tendons gauches constituant les témoins négatifs. L'examen exhaustif des tendons par des coupes transversales se révélait impossible à réaliser car il aurait nécessité plus de 400 coupes par tendon de souris.

L'examen des tendons de 6 souris, par la technique de coloration au X-Gal *in toto,* a permis de voir macroscopiquement une zone bleue à J2 et J8. Des coupes transversales, orientées sur ces zones bleues, permettaient de retrouver des plages de cellules transduites.

En résumé, le transgène a été uniquement retrouvé sur les tendons injectés par le plasmide CMV-LacZ nu, aux 3 délais de J2, J4 et J8, et l'expression du transgène était paratendineuse. Aucune réaction inflammatoire n'a été mise en évidence, ni macroscopiquement, ni histologiquement.

### Comparaison de l'injection de plasmides CMV-LacZ nus et enrobés de PEI

L'influence d'un agent facilitant (PEI) sur le transfert in vivo a été évaluée. Cette étude, effectuée au temps précoce, a analysé les résultats de l'injection de plasmides CMV-LacZ, nus ou enrobés de PEI, dans 4 tendons d'Achille de souris. Les plasmides ont été injectés, sous un même volume (40µl), sous contrôle de la vue après incision cutanée.

Il était apparu, lors des injections, qu'au-delà de 10 µI le liquide refluait hors du tendon. De même, l'aiguille devait rester en place au minimum une minute pour éviter ce reflux lors du retrait de l'aiguille.

La technique de coloration au X-Gal *in toto,* effectuée à 48 H, a montré sur des coupes transversales centrées sur les zones macroscopiquement bleues des tendons, la présence d'une activité β-galactosidase dans la profondeur des tendons (figures 9). La différence d'intensité de transfection, entre plasmides nus et enrobés de PEI, n'a pas pu être quantifiée sur les coupes histologiques. La coloration, également appliquée sur les tissus péritendineux à la recherche d'une diffusion, a montré l'absence de β-galactosidase en position péritendineuse tendons.

### Injection de plasmides CMV-LacZ nus après incision cutanée

La réalisation d'une incision cutanée, lors de l'injection des plasmides, permettait le contrôle visuel de l'injection, comme cela a été montré dans l'étude précédente. Afin de valider cette technique, l'analyse de l'expression du transgène a été réalisée sur les tendons de 3 souris. Ces tendons ont été injectés avec 40 µg de plasmide CMV-LacZ nu du côté droit (sous 40 µI) et avec 40 µg de plasmide non LacZ du côté gauche (sous 40 µl). Le transfert du gène Lac Z a été analysé par coloration au X-Gal *in toto,* c'est à dire sur tout le prélèvement (muscle-tendon et tissus péri-tendineux), à 48 heures. Le résultat macroscopique était très positif sur les 3 tendons droits et négatif sur les tendons gauches servant de contrôle (figures 9). Les zones de transduction, bleues, étaient très limitées et localisées sur le tendon et le paratendon. Les coupes histologiques, de bonne qualité, montraient la présence du transgène à l'intérieur du tendon, prouvant la possibilité de transduction des cellules intra-tendineuses par une technique d'injection adaptée. Dans tous les cas, les zones de transfection étaient localisées, voire limitées à la zone de traumatisme du biseau de l'aiguille (figure 9).

L'intensité d'expression du gène Lac Z a été étudiée à 48 heures de la transfection *in situ* des tendons d'une série de 4 souris. Les tendons étaient injectés, sous contrôle de la vue, avec un volume de 20 µl contenant 40 µg d'ADN (2 µg/µl). Six des huit tendons ont été injectés avec le plasmide CMV-LacZ, les deux autres par un plasmide non LacZ (témoins). Après prélèvement, à 48 heures, les tendons ont été répartis au hasard dans deux groupes, chacun des groupes ayant un tendon témoin. Le 1^{er} groupe a été étudié par la technique de coloration au X-gal *in toto* tandis que le 2^{nd} groupe était étudié en utilisant le kit de chémi-luminescence permettant la quantification de l'activité β-galactosidase. Le but de l'étude par ces 2 techniques, en parallèle, était de vérifier que la technique du kit, analysant uniquement le tendon, s'effectuait sur des tendons réellement transduits comme pouvaient le montrer les études macroscopiques et histologiques réalisées sur l'autre groupe de tendons. La technique de coloration au X-gal *in toto,* de bonne qualité technique, a confirmé la présence de cellules transduites dans les tendons injectés par le plasmide CMV-LacZ alors que le tendon témoin était négatif.

La quantification de l'activité β-galactosidase, par le kit de chémi-luminescence, a montré un taux d'expression proche dans les 3 tendons injectés par le plasmide CMV-LacZ (tableau IV). Chaque mesure a été effectuée 2 fois, la conversion effectuée à partir de la gamme avait un coefficient de détermination R² égal à 0,998, témoignant de la précision du calcul de conversion.

### Injections de plasmides CMV-LacZ nus sous différents volumes et concentrations

Différentes possibilités de formulation des plasmides ont été analysées en vue de l'optimisation du transfert *in situ.* Le but était d'abord d'étudier l'influence des volumes injectés sous une concentration donnée. Afin de conserver une quantité suffisante de plasmides, le volume maximal injectable dans un tendon étant de 10 µI, la concentration utilisée était de 7,25 µg/µl. Différentes conditions de transfection *in vivo* ont été étudiées, 48 heures après l'injection de plasmides CMV-LacZ nus, dans une série de 5 souris. Neuf tendons ont été injectés avec un volume variable, de 0,5 à 8 µI, de plasmides CMV-LacZ. Le dernier tendon, servant de contrôle, a été injecté avec un plasmide non LacZ. Les résultats de ces transfections ont été évalués par mesure de l'activité β-galactosidase en utilisant le kit de chémi-luminescence. Les résultats de deux mesures distinctes, réalisées à des temps différents, montraient la variation des valeurs de chaque échantillon (tableau V).

### Transfection in vivo dans des tendons d'Achille de lapin.

L'étude de la distribution du gène LacZ, après injection intra-tendineuse pure de 100 µg de plasmide CMV-LacZ nu dosé à 1 µg/µl dans deux tendons d'Achille de lapin, a montré l'efficacité du transfert plasmidique. La mise en évidence du transfert a été effectuée par la coloration standard au X-Gal. Les coupes histologiques transversales, réalisées à 24 H, étaient dirigées sur la zone injectée. Le transfert était strictement intra-tendineux et aucune coloration n'était visible en dehors du trajet d'injection. Compte tenu de la grande taille des tendons, quelques coupes seulement ont pu être réalisées.

### Analyse de la tolérance des transfections in vivo.

Lors de l'examen macroscopique des tendons de souris ou de lapin, aucun signe d'inflammation n'a été vu que ce soit au niveau du tendon, des tissus péri-tendineux ou de l'articulation de la cheville.

Lors de l'analyse microscopique, améliorée par la contre-coloration à l'HE, aucune infiltration de cellules mononucléées n'a été vue autour des ténocytes transduits par le gène LacZ.

L'ensemble des résultats obtenus montre donc que le transfert de plasmides in vivo dans les ténocytes est possible, avec de l'ADN nu ou associé à un agent facilitant (type polymère cationique), que ce transfert permet l'expression d'un gène recombinant dans les ténocytes in vivo, et que le transfert n'induit pas d'inflammation autour des cellules transduites ni de modification du volume du tendon.

### EXEMPLE 5: IMPLANTATION IN VIVO DE TENOCYTES MODIFIES GENETIQUEMENT EX VIVO

Des essais de transfert de gène ont été effectués dans des tendons par l'intermédiaire de ténocytes homologues modifiés génétiquement ex vivo (en particulier transduits par des rétrovirus GALV 18).

Un 1er essai a étudié la possibilité de détecter l'expression précoce du transgène, lors de la réimplantation de 5.10⁶ ténocytes transduits. Les essais suivants ont évalué la durée d'expression du transgène par une analyse à différents délais (de 24 heures à 26 jours) après implantation de 5.10⁶ ténocytes transduits. La tolérance du transfert *ex vivo* a été particulièrement étudiée lors d'examens macroscopiques et histologiques.

### 5.1. Modèle animal

Des lapins mâles de type New Zealand, de 4 semaines, pesant 2,5 Kg (ESD 01-France) ont été utilisés. L'anesthésie était assurée par l'utilisation d'Hypnovel® 5 mg (1 ml) administré par voie IM et de Ketalar® (kétamine) à la dose de 80 mg/Kg administré par voie IM. Le sacrifice était réalisé par l'injection IV d'une dose léthale (50 mg) de Nesdonal® (thiopental).

### 5.2. Préparations cellulaires

Les cellules, implantées ou réimplantées, étaient des ténocytes autologues ou homologues (allogéniques). Un nombre variable de ténocytes (de 2.10⁶ à 5.10⁶ cellules), contenus dans un volume de 250 µI d'une solution de DMEM, a été utilisé. Les ténocytes étaient fraîchement infectés, ou issus d'une lignée transduite, par des vecteurs de type GALV 18 véhiculant le gène nls-LacZ.

### 5.3. Injection des cellules

La zone opératoire était préparée en respectant les conditions d'asepsie chirurgicale. L'injection de la préparation cellulaire était effectuée, dans le tendon ou dans sa gaine, parallèlement au tendon et après incision cutanée permettant le contrôle visuel. Une seringue d'un ml, de type « insuline », était utilisée avec une aiguille de 22 G. L'incision cutanée était refermée par une suture simple au fil de nylon.

### 5.4. Résultats

Les essais de transfert ex vivo ont été effectués sur des tendons rotuliens de lapins mâles de type New Zealand, âgés de 4 semaines.

### Etude du transfert au temps précoce

Dans le but d'évaluer la possibilité du transfert de gène *ex vivo,* l'expression du transgène dans des tendons rotuliens sains a été analysée à court terme. La réimplantation, dans chacun des tendons rotuliens d'un lapin, de 5.10⁶ ténocytes homologues a été étudiée à un délai de 24 heures. Parmi ces ténocytes de passage 18 et fraîchement infectés, 60 p.100 étaient transduits. L'implantation de ces ténocytes était effectuée dans des tendons intacts, après incision cutanée épargnant la gaine. Le 1^{er} côté était injecté dans la gaine et le tendon, le 2^{nd} dans le tendon uniquement.

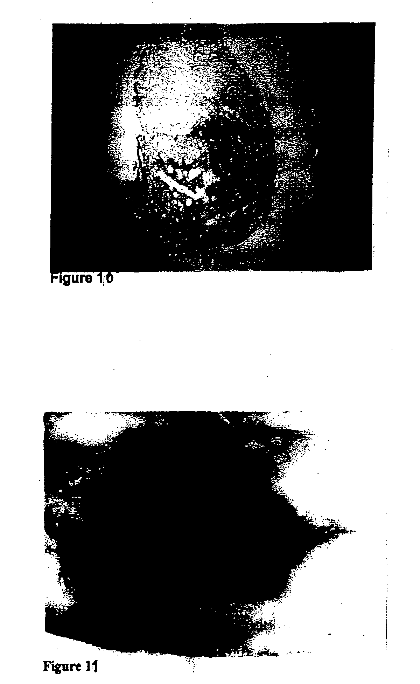

Les analyses macroscopiques et histologiques (coloration sur lames), réalisées à 24 heures, ont montré l'absence d'adhérence cutanée et de réaction inflammatoire (articulaire et extra-articulaire). Le gène marqueur a été très nettement détecté sur les coupes histologiques avec la technique de coloration au X-Gal sur lames (Figure 10).

### Etude de la durée de détection du transgène

### Analyse du transfert de J2 à J8

L'analyse effectuée au temps précoce ayant montré la possibilité de réalisation du transfert *ex vivo* et sa détection par la technique histologique de coloration sur lames, une 2^{nde} étude a consisté à étudier la durée d'expression du transgène dans des tendons intacts de lapin. La réimplantation, dans chacun des tendons rotuliens, de 5.10⁶ ténocytes homologues a été étudiée à un délai de 2, 4 et 8 jours. Parmi ces ténocytes (de passage 28 issus d'une lignée infectée à passage 18), 88 p.100 étaient transduits. L'implantation de ces ténocytes était effectuée à l'intérieur des tendons, après incision cutanée épargnant la gaine. Le 1^{er} côté était injecté avec des ténocytes transduits, le 2^{nd} avec des ténocytes non transduits.

L'analyse macroscopique a montré l'absence d'adhérence cutanée et de réaction inflammatoire (articulaire et extra-articulaire). Un épaississement de la gaine a été constaté jusqu'à 4^{ème} jour.

L'analyse histologique par coloration au X-Gal sur lames a montré la présence du gène marqueur à J2, J4 et J8 (figure 10). Toutes les coupes de tendons ayant reçus des ténocytes transduits montraient la présence du gène marqueur, tandis que les coupes de tendons témoins montraient l'absence de ce gène. Les cellules transduites étaient moins visibles et semblaient dispersées à J8, l'analyse au fort grossissement montrait cependant la persistance du transgène.

L'injection de 5.10⁶ ténocytes homologues a été étudiée dans 4 tendons rotuliens intacts à un délai de 48 heures. Parmi ces ténocytes (de passage 32, issus d'une lignée infectée à passage 18), 89 p.100 étaient transduits. L'implantation de ces ténocytes a été effectuée à l'intérieur de tendons, après incision cutanée épargnant la gaine. Le 1^{er} côté était injecté avec des ténocytes transduits, le 2^{nd} avec des ténocytes non transduits.

La visualisation macroscopique de l'expression du transgène a permis de localiser la zone de transduction sur le tendon et d'orienter les coupes. Les ténocytes transduits ont été trouvés dans le tendon mais aussi dans sa gaine (figure 11). Sur le tendon témoin, aucune transduction n'était visible ni dans la gaine, ni dans le tendon (figures 11). L'étude des tissus péritendineux, à la recherche d'une diffusion du marqueur au-delà de la gaine, a montré l'absence de celui-ci. La technique de coloration *in toto* s'est révélée de bonne qualité histologique et permettant une contre-coloration par l'HE. Les coupes de tendons témoins confirmaient l'absence de transgène Avec la technique du kit de chémi-luminescence, l'activité β-galactosidase, mesurée dans un tendon transduit, était de 4070 pg pour un témoin à 0 pg.

### Analyse du transfert de J8 à J26

Après l'amélioration des méthodes d'analyse du transfert *ex vivo,* il était intéressant d'étudier la durée d'expression du transgène dans des tendons intacts au-delà de 8 jours. La réimplantation de 5.10⁶ ténocytes homologues, par tendon rotulien, a été étudiée à un délai de 8, 14 et 26 jours. Parmi ces ténocytes (de passage 33, issus d'une lignée infectée à passage 18), 94 p.100 étaient transduits. L'implantation était effectuée à l'intérieur des tendons, après incision cutanée épargnant la gaine. Les 2 côtés étaient injectés avec des ténocytes transduits. Les contrôles négatifs de coloration étaient les tendons d'Achille des mêmes lapins injectés par des ténocytes non transduits. Une analyse a été effectuée en parallèle avec du 1^{er} côté, la technique de coloration *in toto,* et du 2^{nd} côté, la quantification de l'activité β-galactosidase par le kit de chémi-luminescence.

L'analyse macroscopique montrait l'absence d'adhérence cutanée et de réaction inflammatoire (articulaire et extra-articulaire). La visualisation macroscopique de l'expression du transgène était possible sur le tendon jusqu'à J14.

L'analyse histologique, par la technique de coloration *in toto,* montrait la persistance du gène marqueur à J8, J14 et J26 sur des coupes centrées sur la zone colorée visible macroscopiquement. Neuf coupes ont été effectuées par tendon. Toutes les coupes de tendons, ayant reçus des ténocytes transduits, montraient la présence du gène marqueur, tandis que celles des tendons témoins montraient l'absence de ce gène. La dispersion dans le tendon des cellules transduites augmentait de J8 à J26. La qualité des coupes par coloration *in toto* était comparable à celle de la technique classique, comme le montrent les lames réalisées à 8 jours de recul. Au dernier délai, des images de réaction à corps étranger étaient visibles autour d'amas de β-galactosidase.

### Analyse de la tolérance du transfert de gènes ex vivo

Lors de l'examen macroscopique des tendons rotuliens après implantation de ténocytes transduits, aucun signe d'inflammation n'a été vu que ce soit au niveau du tendon, des tissus péri-tendineux ou de l'articulation du genou.

Lors de l'analyse microscopique, améliorée par la contre-coloration à l'HE, aucune infiltration de cellules mononucléées n'a été vue autour des ténocytes homologues, transduits ou non, dans les phases précoces.

### RESUME

En résume, les résultats obtenus montrent en particulier que :
- des ténocytes peuvent être isolés à partir de tendons puis cultivés en masse afin d'effectuer leur réimplantation,
- les ténocytes peuvent être transduits très efficacement *in vitro* par l'intermédiaire des vecteurs rétroviraux GALV 18,
- l'expression d'un transgène dans les ténocytes transduits par un vecteur rétroviral est stable en culture au-delà de 45 jours,
- le transfert d'un gène est possible *in vivo,* par l'intermédiaire de plasmides nus ou enrobés de PEI, avec une persistance de l'expression à 8 jours,
- l'expression d'un transgène *in vivo,* après implantation dans les tendons de ténocytes transduits, est encore présente à 26 jours.

### REFERENCES

1.Catonné et al., Catonné Y, Saillant G. ed. Lésions traumatiques des tendons chez le sportif Paris :Masson, 1992 : 3-8.
2.Christel P., J. Traumatol.Sport. 14 (1997) 66
3.Imbert JC, J. Traumatol.Sport. 14 (1997) 107
4.Poddevin et al., Rev.Chir.Orthop.Rappar.Apar.Mot 81 (1995) 410
5.Natsu-ume et al., J. Orthop. Res 15 (1997) 837
6.Panossian et al., Clin. Orthop. 342 (1997) 173
7.Lee et al., Orthop. J. 18 (1998) 19
8. Sambrook et al., Molecula r Clonig, A laboratory Manual, 2nd edition New York: Cold Spring Harbor Lab. Press 1989
9.Miller et al., J . Virol65 (1991) 2220
10. Bernard-Beaubois et al., Cell. Biol. Toxicil. 13 (1997) 103
11. Cao et al., Transplant. Proc. 26 (1994) 3390
12. Kao et al., Arch. Biochem. Biophys. 173 (1976) 638
13.Anselme et al., Rev.Chir.Orthop.Rappar.Apar.Mot 82 (1996) 709

**Tableau I:**

| Temps de doublement des ténocytes de lapin *in vitro.* | | | | | | | | | | |
|---|---|---|---|---|---|---|---|---|---|---|
| | **Temps de doublement (heures)** | | | | | | | | | |
| **N° de repiquage** | **1** | **2** | **3** | **4** | **5** | **6** | **7** | **8** | **9** | **10** |
| **Expérience 1** | 105 | 325 | 95 | 247 | 297 | 220 | 319 | 198 | 121 | 79 |
| **Expérience 2** | 277 | 80 | 58 | 59 | 73 | 54 | 40 | | | |

**Tableau II:**

| Taux d'infection des ténocytes de lapin en fonction de leur taux de confluence et du renouvellement ou non du surnageant viral. | | | |
|---|---|---|---|
| | **surnageant unique** | **surnageant renouvelé 1 fois** | **surnageant renouvelé 2 fois** |
| **25% confluence** | 81,8% | 74,7% | 72,8% |
| **50% confluence** | 79,4% | 73,2% | 71,8% |
| **75% confluence** | 57,7% | 59,7% | 50,9% |

**Tableau III:**

| Evolution du nombre total théorique de ténocytes transduits. | | |
|---|---|---|
| **Délais (jours)** | **Nombre de ténocytes (exp. 1)** | **Nombre de ténocytes (exp. 2)** |
| **0** | 1,2.10⁶ | 1,2.10⁶ |
| **7** | 1,8.10⁶ | 1,8.10⁶ |
| **14** | 3,6.10⁶ | 6,7.10⁶ |
| **21** | 1,2.10⁷ | 7,1.10⁷ |
| **29** | 2,7.10⁷ | 5,8.10⁸ |
| **35** | 4,7.10⁷ | 3,8.10⁹ |
| **42** | 9,8.10⁷ | 4,5.10¹⁰ |
| **49** | 1,0.10⁸ | 5,2.10¹¹ |
| **56** | 2,1.10⁸ | |
| **63** | 6,4.10⁸ | |
| **70** | 2,4.10⁹ | |
| **77** | 1,8.10⁹ | |

**Tableau IV:**

| Taux de β-galactosidase détecté par le kit de chémi-luminescence à 48 heures. | | | | |
|---|---|---|---|---|
| | **Tendon 1** | **Tendon 2** | **Tendon 3** | **Tendon 4** |
| **Plasmide** | CMV-LacZ | CMV-LacZ | CMV-LacZ | non LacZ |
| **Mesure 1 (pg)** | 393,7 | 425,3 | 352,9 | 21,5 |
| **Mesure 2 (pg)** | 361,4 | 399,8 | 341,2 | 17,1 |

**Tableau V:**

| Taux de β-galactosidase lors du transfert *in situ* par le plasmide CMV-LacZ nu. | | | | | | | | | | |
|---|---|---|---|---|---|---|---|---|---|---|
| | **1** | **2** | **3** | **4** | **5** | **6** | **7** | **8** | **9** | **10** |
| Plasmide | LZ | LZ | LZ | LZ | LZ | LZ | LZ | LZ | LZ | No |
| Volume (µI) | 0,5 | 0,5 | 1 | 1 | 2 | 2 | 4 | 4 | 8 | 8 |
| Concentr (µg/µl) | 7,25 | 7,25 | 7,25 | 7,25 | 7,25 | 7,25 | 7,25 | 7,25 | 7,25 | 2 |
| Quantité (µg) | 3,625 | 3,625 | 7,25 | 7,25 | 14,5 | 14,5 | 29 | 29 | 58 | 16 |
| Mesure 1 (pg) | 6,6 | 8,8 | 9,7 | 9,5 | 35,5 | 44,1 | 58,6 | 100,0 | 124,6 | 13,9 |
| Mesure 2 (pg) | 16,4 | 26,4 | 21,4 | 26,7 | 79,5 | 44,0 | 89,2 | 99,7 | 227,2 | 29,6 |
| LZ : LacZ Note: Réalisation de 2 mesures (1 et 2), à des temps différents. | | | | | | | | | | |

## Revendications

1. Composition, caractérisée en ce qu'elle comprend des cellules ténocytes autologues et/ou allogéniques d'origine humaine.

2. Composition selon la revendication 1, caractérisée en ce qu'il s'agit d'une culture in vitro ou ex vivo de ténocytes autologues et/ou allogéniques.

3. Composition selon la revendication 1 ou 2, caractérisée en ce qu'il s'agit de ténocytes humains autologues et/ou allogéniques modifiés génétiquement.

4. Composition selon l'une des revendications 1 à 3, comprise dans un dispositif de type ampoule, seringue, fiole, poche ou boite.

5. Composition selon l'une des revendications 1 à 4, caractérisée en ce qu'il s'agit de ténocytes préparés à partir de tendon.

6. Composition selon la revendication 5, caractérisée en ce qu'il s'agit de ténocytes préparés à partir du tendon d'un muscle de la région patte d'oie, du tendon du muscle petit palmaire, du tendon du muscle plantaire grêle, du tendon du muscle péronier antérieur, du tendon quadricipital, du tendon rotulien ou du tendon d'Achille.

7. Composition selon l'une des revendications 1 à 4, caractérisée en ce qu'il s'agit de ténocytes préparés à partir de ligament.

8. Composition selon l'une des revendications 1 à 4, caractérisée en ce qu'il s'agit de ténocytes préparés à partir d'une aponévrose.

9. Composition, caractérisée en ce qu'elle comprend des ténocytes humains ou équins modifiés génétiquement et un excipient.

10. Composition selon la revendication 9, caractérisée en ce qu'elle comprend des ténocytes humains modifiés génétiquement.

11. Cellule ténocyte isolée et modifiée génétiquement, caractérisée en ce qu'elle contient un acide nucléique recombinant codant un facteur de croissance et/ou un facteur inhibant la réaction inflammatoire.

12. Composition comprenant des ténocytes congelés.

13. Composition pour la conservation de ténocytes, comprenant des ténocytes et au moins un composé choisi parmi :
a. du diméthyl sulfoxyde
b. une gélatine modifiée
c. un polysaccharide, ou
d. du glycérol.

14. Utilisation de ténocytes humains pour la préparation d'une composition destinée à l'implantation in vivo.

15. Utilisation, selon la revendication 14, de ténocytes pour la préparation d'une composition destinée à la mise en oeuvre d'une méthode de traitement thérapeutique, diagnostique ou chirurgical du corps humain.

16. Utilisation selon la revendication 15, caractérisée en ce qu'il s'agit de ténocytes autologues, allogéniques ou xénogéniques.

17. Utilisation selon l'une des revendications 14 à 16 caractérisée en ce qu'il s'agit de ténocytes cultivés in vitro.

18. Utilisation selon l'une des revendications 14 à 17 caractérisée en ce qu'il s'agit de ténocytes modifiés génétiquement.

19. Utilisation selon la revendication 18, caractérisée en ce qu'il s'agit de ténocytes contenant un acide nucléique recombinant codant un facteur de croissance et/ou un facteur inhibant la réaction inflammatoire.

20. Procédé de modification génétique d'un ténocyte in vitro ou ex vivo, comprenant la mise en contact d'un ténocyte avec (i) de l'ADN nu ou (ii) un rétrovirus recombinant pseudotypé avec une enveloppe GALV.

21. Utilisation d'une composition comprenant (i) de l'ADN nu ou (ii) un rétrovirus recombinant pseudotypé avec une enveloppe GALV, pour la préparation d'une composition destinée au transfert de cet ADN dans les ténocytes in vivo.

22. Utilisation d'un acide nucléique recombinant pour la préparation d'une composition destinée à la modification génétique in vivo d'un ténocyte humain.

23. Utilisation, selon la revendication 14, d'une population de ténocytes ou de fibroblastes, autologues allogéniques ou xénogéniques, pour la préparation d'une composition destinée à l'implantation ou l'administration dans un tendon ou ligament.

24. Utilisation selon la revendication 14, d'une population de ténocytes ou de fibroblastes, autologues allogéniques ou xénogéniques, pour la préparation d'une composition destinée à favoriser la cicatrisation d'une déchirure ou rupture musculaire.

25. Procédé de production de ténocytes, comprenant :
- le prélèvement d'un fragment de tissu biologique comprenant des ténocytes, de préférence d'origine humaine,
- le traitement de ce fragment pour dissocier les ténocytes, en présence d'une collagénase, et
- la mise en culture des ténocytes, et leur multiplication in vitro par passages successifs, de préférence par ensemencement à une densité correspondant au quart de la confluence environ.

26. Procédé selon la revendication 25, comprenant une étape de congélation des ténocytes, de préférence dans un milieu selon la revendication 13.

27. Procédé selon la revendication 25 ou 26, comprenant une étape de modification génétique des ténocytes, de préférence selon la revendication 20.
